# EUROPEAN PATENT SPECIFICATION

(11) **EP 3 403 626 B1**
(45) Date of publication and mention of the grant of the patent: **09.10.2024**
(21) Application number: 17171110.4
(22) Date of filing: 15.05.2017
(51) Int. Cl.: A61F 13/475, A61F 13/49, A61F 13/511, A61F 13/534

(54) **ABSORBENT ARTICLE WITH CHANNELS AND METHOD FOR MANUFACTURING THEREOF**
SAUGFÄHIGER ARTIKEL MIT KANÄLEN UND HERSTELLUNGSVERFAHREN DAFÜR
ARTICLE ABSORBANT COMPRENANT DES CANAUX ET SON PROCÉDÉ DE FABRICATION

(43) Date of publication of application: 21.11.2018
(73) Proprietor: Drylock Technologies NV, 9240 Zele (BE)
(72) Inventor: SMET, Steven, 9240 Zele (BE); VAN INGELGEM, Werner, 9240 Zele (BE); DERYCKE, Tom, 9240 Zele (BE)
(74) Representative: D'Halleweyn, Nele Veerle Trees Gertrudis

(56) References cited:
- EP-A1- 2 949 302
- EP-B1- 3 342 386
- WO-A1-2015/031225
- US-A1- 2014 163 503
- US-A1- 2014 371 701

## Description

### TECHNICAL FIELD

The present invention pertains to the technical field of absorbent articles, more preferably disposable personal care articles such as diapers, baby pants, adult incontinent garments, and the like, and to absorbent structures for use in such absorbent articles. More specifically the present invention relates to an absorbent structure comprising an absorbent core between a topsheet and a backsheet. The present invention also relates to a method for manufacturing such an absorbent article.

### BACKGROUND

Absorbent articles such as diapers, baby pants, adult incontinent garments and the like, typically comprise an absorbent core, positioned in between a liquid permeable or pervious, hydrophilic or semi hydrophilic topsheet and a liquid impermeable or impervious backsheet. The absorbent core comprises absorbent material that is able to absorb fluid and liquid bodily excretions of the user of the absorbent article.

The absorbent material of the absorbent core may be an absorbent particulate polymer material which is dispersed in a matrix of cellulose fibers or fluff pulp in order to prevent the particulate material from aggregating, as well as to prevent gel blocking. Gel blocking can occur when the absorbent particulate polymer material absorbs liquid, as they tend to typically swell and form a gel structure. This gel structure often blocks the further transfer of liquid into the remaining absorbent core. As a result, the liquid may be unable to reach the remaining absorbent particulate polymer material and the efficiency of the overall absorbent article decreases significantly. Existing fluff pulp materials are not suited to cope with rapid, subsequent insults of fluid since they possess limited distribution capacities. Moreover existing fluff pulp materials exhibit a limited capacity of overall liquid intake. Furthermore, existing absorbent cores containing fluff pulp have a limited wet integrity, which leads to the shape and fit of the absorbent article being deformed when e.g. an absorbent article is being worn by a baby which moves around.

In recent years, there has been a strong demand for more flexible, thinner, light-weight, absorbent articles to resolve various problems associated with manufacturing, marketing, design, fit, wearing comfort, distribution, garbage disposal, material and energy consumption, transport and storage costs and the like. This lead to the search for and the development and production of absorbent articles of which the absorbent cores contains little to no cellulose fibers or fluff pulp, as the latter tend to be quite bulky, thus rendering generally more thick absorbent cores which reduces the overall wearing comfort of the user of the absorbent article.

Hence, various absorbent cores containing little to no cellulose fibers or fluff pulp were developed in the past few years to try and overcome the above drawbacks, whereby the relative high amounts of absorbent polymer materials necessary to replace the absorption, distribution and retention capacity of the excluded cellulose fibers and/or fluff pulp were loaded, distributed and immobilized within these new absorbent cores according to several techniques. However given the ability and capacity of the absorbent core to absorb, transport and retain fluid and liquids is heavily dependent upon the form, position and/or manner wherein these absorbent polymer materials are incorporated within the absorbent core several drawback remained unsolved. In general the substantially heterogeneously distributed absorbent cores having non-continuous compartments and/or clusters of absorbent polymer material have in general proven to be better in coping with the above mentioned problems, nevertheless they also proved to remain unsatisfactory within most of the available absorbent articles. Especially problematic however, were the substantially homogenously distributed absorbent structures having continuous layers of absorbent polymer particulate material given they exhibit a substantially homogenous swollen absorbent polymer material area for second, third and next liquid insults wherein the dry and/or wetted absorbent polymer material layer may actually act as a liquid barrier. These problems and complications are especially prevalent within very flexible, thin, lightweight absorbent structures wherein high amounts of absorbent polymer material are distributed within the absorbent core of the absorbent article. Adding even more, thicker and larger overlying acquisition and dispersing layers did not at all resolve the above cited absorption, distribution and retention problems and moreover made the absorbent articles commercially unviable, environmentally unsustainable and more difficult to manufacture, store and transport.

Furthermore an existing problem which has been associated with such absorbent cores containing no or little cellulose fibers or fluff pulp is related to the migration, loss and leakage of the absorbent particulate polymer material from the absorbent article during dry and/or wet state, which leads to irritation, skin problems and overall discomfort for the user. This again is also especially true in the more homogenously distributed absorbent structures given their immobilization and liquid distribution properties remain unsatisfactory to date. This lack of effective and efficient immobilization and liquid distribution lead to dysfunctional absorbent articles due to lowered uptake capacity, gel blocking, enhanced rewet values, leakages and the creation of ruptures and/or pinholes through the liquid pervious topsheet and/or liquid impervious backsheet of such absorbent articles.

Absorbent cores generally have a high absorbent capacity and the absorbent core may expand several times its weight and volume. These increases may cause the absorbent article to deform and/or to sag in the crotch region as they become saturated with liquid. This may cause leaks to occur via a longitudinal and/or transversal edge of the absorbent article.

US 2014/0163503 discloses an absorbent article having an absorbent core comprising a core wrap enclosing an absorbent material, which comprises at least 80% of superabsorbent polymers by weight. The absorbent core further comprises at least one channel and an acquisition-distribution system (ADS) between the topsheet and the absorbent core, the ADS comprising one, two or more layers wherein the ADS does not comprise a layer comprising at least 50% by weight of synthetic fibers and having a basis weight above 150 gsm.

### SUMMARY

The object of embodiments of the invention is to provide an absorbent article of the type stated in the preamble, with improved liquid distribution and absorption capacities.

According to a first aspect of the invention there is provided an absorbent article according to claim 1. Upon wetting of the absorbent material of the absorbent article, any one of the in claim 1 described conditions leads to the creation of a first and second channel at the first and second attachment zone, respectively.

Embodiments are based *inter alia* on the inventive insight that, by providing a plurality of attachment zone in the absorbent core, a corresponding plurality of channels is created in the absorbent core upon wetting such that liquid can be distributed and absorbed in an improved manner. Indeed, liquid can flow in the plurality of attachment zones and can be absorbed by the absorbent core through the side walls of the plurality of attachment zones, in addition to liquid being absorbed through the top surface of the absorbent core. Because the first and second attachment zones extend in the direction of the first and/or second transverse edge as do the created first and second channel, liquid can be distributed adequately. Both the plurality of attachments zones, before swelling of the absorbent material, and the plurality of created channels, during and after swelling of the absorbent material, allow for a more rapid distribution of liquid, especially towards the transverse edges of the absorbent core. In addition to a fast and adequate distribution of liquid in the longitudinal direction, the presence of the plurality of attachment zones and/or the creation of the corresponding plurality of channels leads to a more rapid and efficient distribution of liquid in both the transverse direction of the absorbent core and in the depth direction of the absorbent core. Furthermore, overall liquid intake by the absorbent core is faster as a result. By giving the attachment zones a sufficient width, depth and/or length a quantity of liquid can be held temporarily whilst the absorption takes place. Because the liquid is distributed quickly, this effect is established not only during a first liquid insult, but also during an eventual second liquid insult, a third liquid insult and a fourth liquid insult. Further, the first and second attachment zones allow the absorbent core to swell in the shape of a tub while the first and second channels are formed. Indeed, a portion of the absorbent core between the first longitudinal edge and the first attachment zone will be allowed to rotate inward and upward and a portion of the absorbent core between the second longitudinal edge and the second attachment zone will be allowed to rotate inward and upward, which is made possible thanks to the sufficiently wide first and second attachment zone.

In an exemplary embodiment, the attachment between the top core wrap sheet and the back core wrap sheet in the first and the second attachment zone is a permanent attachment, and the absorbent core is configured such that, in a wetted state of the absorbent material, the absorbent material extends over the first and second attachment zone. In that matter, the absorbent material bulges over the first and second attachment zone, thereby causing a tension in the absorbent core which causes the absorbent core, which is in a substantially flat state when dry, to curl up to form a tub shaped and/or cup shaped absorbent core including the first and second channel.

Preferably, the plurality of attachment zones cover together at least 30 %, preferably at least 40%, preferably at least 50%, more preferably at least 60%, more preferably at least 70%, more preferably 80% and more preferably at least 90% of a total length of the absorbent core. The covered length may be realized with the first and second attachment zone alone, or with a combination of a first and second attachment zone and one or more additional attachment zones. For example, first and second adjacent longitudinal attachment zones together with third and fourth adjacent longitudinal attachment zones may extend over at least 30 %, preferably at least 40%, preferably at least 50%, more preferably at least 60%, more preferably at least 70%, more preferably 80% and more preferably at least 90%of a total length of the absorbent core. This will allow a good distribution over the entire absorbent core as well as a good formation of the channels and the tub-shape upon swelling of the absorbent core.

According to a preferred embodiment, outside of the plurality of attachment zones the absorbent core has a maximum thickness; wherein the first and second attachment zone extend through at least 90 % of the maximum thickness of the absorbent core, more preferably through 100% of the thickness of the absorbent core such that in the first and second attachment zone substantially no absorbent material is present between the top core wrap sheet and the back core wrap sheet.

According to an exemplary embodiment the first attachment zone and the second attachment zone are arranged symmetrically with respect to a longitudinal center line of the absorbent core extending between the first and second transverse edge.

According to a preferred embodiment, the attachment between the top core wrap sheet and the back core wrap sheet is any one of the following or a combination thereof: pressure bonding, thermal bonding, sonic bonding, chemical bonding, adhesive.

According to a preferred embodiment the plurality of attachment zones further comprises a third and a fourth attachment zone located at a distance of each other, the third and fourth attachment zone each extending in the direction of the first and/or second transverse edge.

Preferably, the distance between the first and the second attachment zone is different from the distance between the third and the fourth attachment zone.

According to an exemplary embodiment, the absorbent core has a front portion extending at one side of a transverse crotch line and a rear portion extending at the other side of the transverse crotch line. The first and second attachment zone extend at least in the front portion of the absorbent core; and the third and fourth attachment zone extend at least in the rear portion of the absorbent core.

Preferably, the distance between the first and the second attachment zone is smaller than the distance between the third and the fourth attachment zone.

In a preferred embodiment, the first attachment zone is connected to the third attachment zone through a first transverse attachment zone, and the second attachment zone is connected to the fourth attachment zone through a second transverse attachment zone.

Preferably, the first and the second attachment zone extend in a longitudinal direction of the absorbent core over a length which is longer than the length of the third and fourth attachment zone, and the first and the second attachment zone are located between the third and fourth attachment zone.

In an exemplary embodiment, the third attachment zone and the fourth attachment zone are arranged symmetrically with respect to a longitudinal center line of the absorbent core extending between the first and second transverse edge.

In a preferred embodiment the distance between the first and the second attachment zone is between 10 mm and 50 mm, preferably between 15 mm and 30 mm.

According to an exemplary embodiment, the length of the first and the second attachment zone is larger than 60 mm, preferably larger than 70 mm.

According to an embodiment, the absorbent material comprises cellulosic fluff pulp.

According to an alternative embodiment, the absorbent material is substantially fluffless.

In a preferred embodiment substantially no absorbent material is present in the first and second attachment zone.

According to exemplary embodiment, the first and second attachment zone each have a bottom and a top, wherein the top core wrap sheet is attached to the back core wrap sheet at said bottom, at said top, or between said bottom and said top.

The skilled person will understand that the herein described technical considerations and advantages for absorbent article embodiments also apply to the below described method embodiments, mutatis mutandis.

According to another aspect, there is provided a method for manufacturing an absorbent article according to claim 20.

In a preferred embodiment, the attaching is done by applying pressure and heat on the top core wrap sheet material and/or the back core wrap sheet material in the areas where substantially no absorbent material is present.

According to a further embodiment, the attaching is done by a rotating member which is provided with at least a first and a second seal rib dimensioned for applying pressure and heat on the top core wrap sheet material and/or the back core wrap sheet material in the areas where substantially no absorbent material is present in order to create the first and second attachment zone, respectively.

### BRIEF DESCRIPTION OF FIGURES

The accompanying drawings are used to illustrate presently preferred non-limiting exemplary embodiments of devices of the present invention. The above and other advantages of the features and objects of the invention will become more apparent and the invention will be better understood from the following detailed description when read in conjunction with the accompanying drawings, in which:
Figure 1A is a perspective view of an exemplary embodiment of a diaper, which does not fall within the scope of the claims;
Figure 1B is a top plan view of the diaper of figure 1A;
Figure 1C is a schematic cross-section along line C-C of figure 1B;
Figure 1D is a schematic cross-section along line D-D of figure 1B;
Figure 2 is a perspective view of another exemplary embodiment of a diaper which does not fall within the scope of the claims;
Figure 3A and 3B are cross-sectional views illustrating the effect of liquid being absorbed by the absorbent core of an exemplary embodiment of an absorbent article; and
Figure 4 illustrates schematically an exemplary embodiment of a method and apparatus for manufacturing an absorbent article, the apparatus not being within the scope of the claims;
Figure 4A shows a cross section of an insert placed at a non-suction zone of the exemplary embodiment of figure 4;
Figure 4B shows a top view indicating how inserts may be positioned in order to create non-suction zones for the exemplary embodiment of figure 4;
Figure 4C shows a cross section of the absorbent core when the second sheet 120 is being applied;
Figure 4D shows a cross section of the absorbent core before attaching the first sheet 110 to the second sheet 120;
Figure 5A shows a top view of an exemplary embodiment of an absorbent core with four attachment zones using a first exemplary embodiment of a sealing pattern;
Figure 5B shows a top view of an exemplary embodiment of an absorbent core with four attachment zones using a second exemplary embodiment of a sealing pattern;
Figure 5C shows a top view of an exemplary embodiment of an absorbent core with four attachment zones using a third exemplary embodiment of a sealing pattern;
Figure 5D illustrates a fourth exemplary embodiment of a possible sealing pattern;
Figure 5E illustrates a fifth exemplary embodiment of a possible sealing pattern;
Figure 6 is a perspective view of an exemplary embodiment of a diaper in a wetted state;
Figures 7A and 7B are cross-sectional views illustrating the effect of liquid being absorbed by a traditional absorbent core and liquid being absorbed by an absorbent core according to an exemplary embodiment of the invention, respectively;
Figure 8 illustrates a schematic cross-section of an absorbent core, wherein three possible locations are indicated for the attachment zones; and
Figures 9-12 illustrate exemplary embodiments of an absorbent core according to the invention.

### DESCRIPTION OF EMBODIMENTS

As used herein, the following terms have the following meanings:
"A", "an", and "the" as used herein refers to both singular and plural referents unless the context clearly dictates otherwise. By way of example, "an edge barrier" refers to one or more than one edge barrier.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or variations are appropriate to perform in the disclosed invention. However, it is to be understood that the value to which the modifier "about" refers is itself also specifically disclosed.

"Absorbent article", "absorbent garment", "absorbent product", "absorbing article", "absorbing garment", "absorbing product" and the like as used herein are used interchangeably and refer to devices that absorb and contain bodily exudates, and more specifically, refers to devices that are placed against or in proximity to the body of the wearer to absorb and contain the various liquids discharged from the body. Absorbent articles include but are not limited to feminine hygiene garments, baby diapers and pants, adult incontinence garments, various diaper and pants holders, liners, towels, absorbent inserts and the like.

"Absorbent core" as used herein refers to a three-dimensional part of the absorbent structure, comprising liquid-absorbing material, useful to permanently absorb and/or retain bodily exudates.

"Absorbent component" as used herein refers to a structural constituent of an absorbent article, e.g., a piece of an absorbent core, such as one of multiple pieces in a multi-piece absorbent core.

"Absorbent element" as used herein refers to a part of a functional constituent of an absorbent structure, e.g., a acquisition layer, a dispersion layer, core layer or a release structure formed of a material or materials having particular liquid handling characteristics suitable for the specific function.

"Absorbent fibrous polymer material" as used herein refers to an absorbent polymer material which is in threadlike from such as fibers, filaments, and the like so as to be less flowable in the dry state than particulates.

"Absorbent insert" as used herein refers to a device adapted for insertion into an "Absorbent layer" as used herein refers to a term referring to a discrete, identifiable sheet-like or web-like element of an absorbent article which may remain detached and relatively movable with respect to another such element or may be attached or joined so as to remain permanently associated with another such element. Each absorbent layer may itself include a laminate or combination of several layers, sheets and/or webs of similar or diverse compositions.

"Absorbent polymer material", "absorbent gelling material", "AGM", "superabsorbent", "superabsorbent material", "super absorbent polymer", "SAP" and the like as used herein are used interchangeably and refer to any suitable particulate (e.g., flaked, particulate, granular, or powdered) or fibrous cross linked polymeric materials that can absorb at least 5 times and preferably at least about 10 times or more its weight of an aqueous 0.9% saline solution as measured using the Centrifuge Retention Capacity test (EDANA 441.2-01).

"Absorbent polymer material area" as used herein refers to the area of the absorbent structure wherein adjacent layers are separated by a multiplicity of absorbent polymer material. Incidental contact areas between these adjacent layers within the absorbent particulate polymer material area may be intentional (e.g bond area's) or unintentional (e.g. manufacturing artifacts).

"Absorbent particulate polymer material" as used herein refers to an absorbent polymer material which is in particulate form such as powders, granules, flakes and the like so as to be flowable in the dry state.

"Absorption" as used herein refers to the process by which a liquid is taken up within a material.

"Absorption rate" as used herein refers to the rate of absorption of liquid, i.e. the amount of liquid which is absorbed per unit of time, typically by an absorbent component, element and/or absorbent layer of the absorbent article, structure and/or core.

"Acquisition layer", "acquisition region", "acquisition surface" or "acquisition material" and the like as used herein refer to the layer overlying the absorbent core having a faster liquid uptake and/or distribution capability.

"Absorbency" is the ability of a material to take up fluids by various means including capillary, osmotic, solvent, chemical and/or other action.

"Adult incontinence garment" as used herein refers to absorbent articles intended to be worn by incontinent adults, for absorbing and containing bodily exudates.

"Adhesion" as used herein refers to the force that holds different materials together at their interface.

"Adhesive" as used herein refers to a material, which may or may not be flowable in solution or when heated, that is used to bond materials together.

"Adsorption" as used herein refers to the process by which a liquid is taken up by the surface of a material.

"Airlaying" as used herein refers to forming a web by dispersing fibers or particles in an air stream and condensing them from the air stream onto a moving screen by means of a pressure and/or vacuum; a web of fibers produced by airlaying is herein referred to an "airlaid"; an airlaid web bonded by one or more techniques to provide fabric integrity is herein referred to an "airlaid nonwoven".

"Apparent density", "density" as used herein refers to the basis weight of the sample divided by the caliper with appropriate unit conversions incorporated therein. Apparent density used herein has the unit g/cm³.

"Attach", "attached" and "attachment" as used herein are synonymous with their counterparts of the terms "fasten", "affix", "secure", "bind", "join" and "link".

"Baby diaper" as used herein refers to absorbent articles intended to be worn by children, for absorbing and containing bodily exudates which the user draws up between the legs and fastens about the waist of the wearer.

"Baby pants" as used herein refers to absorbent articles marketed for use in transitioning children from diapers to underwear intended to cover the lower torso of children, so as to absorb and contain body exudates which article is generally configured like a panty garment and manufactured with a completed waist encircling portion, thereby eliminating the need for the user to fasten the article about the waist of the wearer.

"Back region" as used herein refers to the portion of an absorbent article or part thereof that is intended to be positioned proximate the back of a wearer.

"Backing" as used herein refers to a web or other material that supports and reinforces the back of a product.

"Basis weight" is the weight per unit area of a sample reported in grams per square meter, g/m² or gsm.

"Bodily exudates", "body exudates", "bodily fluids", "body fluids", "bodily discharges", "body discharges", "fluid(s)", " liquid(s)", "fluid(s) and liquid(s) and the like as used herein are used interchangeably and refer to, but are not limited to urine, blood, vaginal discharges, breast milk, sweats and fecal matter.

"Binder", "adhesive", "glue", "resins", "plastics" and the like as used herein are used interchangeably and refer to substances, generally in a solid form (e.g. powder, film, fiber) or as a foam, or in a liquid form (e .g. emulsion, dispersion, solution) used for example by way of impregnation, spraying, printing, foam application and the like used for attaching or bonding functional and/or structural components, elements and materials, for example including heat and/or pressure sensitive adhesives, hot-melts, heat activated adhesives, thermoplastic materials, chemical activated adhesives/solvents, curable materials and the like.

"Bond strength" as used herein refers to the amount of adhesion between bonded surfaces. It is a measure of the stress required to separate a layer of material from the base to which it is bonded.

"Capillary action", "capillarity", or "capillary motion" and the like as used herein are used to refer to the phenomena of the flow of liquid through porous media.

"Chassis" as used herein refers to a foundational constituent of an absorbent article upon which the remainder of the structure of the article is built up or overlaid, e.g., in a diaper, the structural elements that give the diaper the form of briefs or pants when configured for wearing, such as a backsheet, a topsheet, or a combination of a topsheet and a backsheet.

"Cellulose fibers" as used herein refers to naturally occurring fibers based on cellulose, such as, for example cotton, linen, etc; wood pulp fibers are one example of cellulose fibers; man-made fibers derived from cellulose, such as regenerated cellulose (rayon), or partially or fully acetylated cellulose derivatives (e.g. cellulose acetate or triacetate) are also considered as cellulose fibers.

"Cluster" or the like as used herein refers to an agglomeration of particles and/or fibers.

"Chemically stiffened fibers", chemically modified fibers", "chemically cross-linked fibers", "curly fibers" and the like as used herein are used interchangeably and refer to any fibers which have been stiffened by chemical means to increase stiffness of the fibers under both dry and aqueous conditions, for example by way of addition of chemical stiffening agents (e.g. by coating, impregnating, etc), altering the chemical structure of the fibers themselves (e.g. by cross-linking polymer chains, etc) and the like.

"Cohesion" as used herein refers to the resistance of similar materials to be separated from each other.

"Compartment" as used herein refers to chambers, cavities, pockets and the like.

"Comprise," "comprising," and "comprises" and "comprised of" as used herein are synonymous with "include", "including", "includes" or "contain", "containing", "contains" and are inclusive or open-ended terms that specify the presence of what follows e.g. a component and do not exclude or preclude the presence of additional, non-recited components, features, elements, members, steps, known in the art or disclosed therein.

"Coverstock" as used herein refers to a lightweight non-woven material used to contain and conceal an underlying absorbent core material; examples are the facing layer or materials that cover the absorbent cores of feminine hygiene garment s, baby diapers and pants and adult incontinence garments.

"Crotch region" of an absorbent article as used herein refers to about 50% of the absorbent article's total length (i.e., in the y-dimension), where the crotch point is located in the longitudinal center of the crotch region. That is, the crotch region is determined by first locating the crotch point of the absorbent article, and then measuring forward and backward a distance of 25% of the absorbent article's total length.

"Cross direction (CD)", "lateral" or "transverse" and the like as used herein are used interchangeably and refer to a direction which is orthogonal to the longitudinal direction and includes directions within ±45° of the transversal direction.

"Curing" as used herein refers to a process by which resins, binders or plastics are set into or onto fabrics, usually by heating, to cause them to stay in place; the setting may occur by removing solvent or by cross-linking so as to make them in soluble.

"Diaper", "conventional diaper", "diaper-like", "diaper-like garment" and the like as used herein are used interchangeably and refer to disposable absorbent articles, which typically include a front waist portion and a back waist portion which may be releasable connected about the hips of the wearer during use by conventional fasteners such as adhesive tape fasteners or hook and loop type fasteners. In use, the article is positioned between the legs of the wearer and the fasteners are releasable attached to secure the back waist portion to the front waist portion of the diaper, thereby securing the diaper about the waist of the wearer. The front waist portion and a back waist portion are connected by relatively non-stretchable or stretchable members (the term "stretchable" as used herein refers to materials that are extensible when forces are applied to the material, and offer some resistance to extension). Hence, such articles are generally not configured to be pulled up or down over the hips of the wearer when the fasteners are attached.

"Dispersion layer", "dispersion region", "dispersion surface" or "dispersion material" and the like as used herein refer to the layer overlying the absorbent core having a faster liquid uptake and dispersion capability.

"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).

"Drylaying" as used herein refers to a process for making a nonwoven web from dry fiber; these terms apply to the formation of carded webs, as well as to the air laying formation of random webs; a web of fibers produced by drylaying is herein referred to as a "drylaid"; a drylaid web bonded by one or more techniques to provide fabric integrity is herein referred to a "drylaid nonwoven".

"Dry strength" as used herein refers to the strength of ajoint determined in dry state conditions, immediately after drying under specified conditions or after a period of conditioning in the standard laboratory atmosphere.

"Essentially cellulose free" or "little to no cellulose fibers" as used herein refers to an absorbent article, structure, core component and/or element containing less than 20% by weight cellulosic fibers, less than 10% cellulosic fibers, less than 5% cellulosic fibers, no cellulosic fibers, or no more than an immaterial amount of cellulosic fibers which do not materially affect the thinness, flexibility or absorbency thereof.

"Essentially fluffless" or "little to no fluff pulp" as used herein refers to an absorbent article, structure, core, component and/or element containing less than 20% by weight fluff pulp, less than 10% fluff pulp, less than 5% fluff pulp, no fluff pulp, or no more than an immaterial amount of fluff pulp which do not materially affect the thinness, flexibility or absorbency thereof.

"Fabric" as used herein refers to a sheet structure made from fibers, filaments and/or yarns.

"Feminine hygiene garments" as used herein refer to absorbent hygiene articles intended to be worn by woman, for absorbing and containing body exudates.

"Fiber" as used herein refers to the basic threadlike structure from which nonwovens, yarns and textiles are made. It differs from a particle by having a length at least 4 times its width; "Natural fibers" are either of animal (wool, silk), vegetable (cotton, flax, jute) or mineral (asbestos) origin, while "Man-made fibers" may be either polymers synthesized from chemical compounds (polyester, polypropylene, nylon, acrylic etc.) or modified natural polymers (rayon, acetate) or mineral (glass). "Fiber" and "filament" are used interchangeably.

"Fluff pulp" or "Pulp fluff" as used herein refers to wood pulp specially prepared to be drylaid. The fibers can be either natural or synthetic or a combination thereof.

"Front region" as used herein refers to the portion of an absorbent article or part thereof that is intended to be positioned proximate the front of a wearer.

"Garment facing layer" as used herein refers to elements of the chassis that form the outer surface of the absorbent article, such as the backsheet, the side panels, the waist fasteners, and the like, when such elements are present.

"Heat activated adhesive" as used herein refers to a dry adhesive that is rendered tacky or fluid by application of heat or heat and pressure to the assembly.

"Heat sealing adhesive" as used herein refers to a thermoplastic adhesive which is melted between the adherent surfaces by heat application to one or both of the adjacent adherent surfaces.

"High loft" as used herein refers to general term of low density, thick or bulky fabrics.

"Hot-melt adhesive" as used herein refers to a solid material that melts quickly upon heating, then sets to a firm bond upon cooling; used for almost instantaneous bonding.

"Hydrophilic" as used herein refers to having an affinity for being wetted by water or for absorbing water.

"Hydrophobic" as used herein refers to lacking the affinity for being wetted by water or for absorbing water.

"Immobilization layer" as used herein refers to a layer able to be applied to the absorbent polymer material or absorbent polymer material area with the intent to gather, bond and/or immobilize absorbent material and/or absorbent layer.

"Join", "joined" and "joining" as used herein refers to encompassing configurations wherein an element is directly secured to another element by affixing the element directly to the other element, as well as configurations wherein the element is indirectly secured to the other element by affixing the element to an intermediate member or members which in turn is or are affixed to the other element.

"Knitting" as used herein refers to the technique for interlocking loops of fibers with needles or similar devices.

"Layer" refers to identifiable components of the absorbent article, and any part referred to as a "layer" may actually comprise a laminate or combination of several sheets or webs of the requisite type of materials. As used herein, the term "layer" includes the terms "layers" and "layered." "Upper" refers to the layer of the absorbent article which is nearest to and/ or faces the wearer facing layer; conversely, the term "lower" refers to the layer of the absorbent article which is nearest to and/or faces the garment facing layer. "Layer" is three dimensional structure with a x dimension width, y dimension length, and z-dimensions thickness or caliper, said x-y dimensions being substantially in the plane of the article, however it should be noted that the various members, layers, and structures of absorbent articles according to the present invention may or may not be generally planar in nature, and may be shaped or profiled in any desired configuration .

"Machine direction (MD)", "longitudinal" and the like as used herein are used interchangeably and refer to a direction running parallel to the maximum linear dimension of the structure and includes directions within ±45° of the longitudinal direction.

"Major surface" as used herein refers to a term used to describe the surfaces of greatest extent of a generally planar or sheet-like structural element and to distinguish these surfaces from the minor surfaces of the end edges and the side edges, i.e., in an element having a length, a width, and a thickness, the thickness being the smallest of the three dimensions, the major surfaces are those defined by the length and the width and thus having the greatest extent.

"Mass flow" as used herein refers to the f low of a liquid from one absorbent element or component to another absorbent element or component by channel flow action.

"Mechanical bonding" as used herein refers to a method of bonding fibers by entangling them. This can be achieved by needling, stitching with fibers or by the use of high-pressure air or water jets and the like.

"Nonwoven" as used herein refers to manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as melt blowing, spun bonding, solvent spinning, electrospinning, and carding. The basis weight of nonwoven fabrics is usually expressed in grams per square meter (gsm).

"Pant", "training pant", "closed diapers", "prefastened diapers", "pull-on diapers" and "diaper-pants" and the like as used herein are used interchangeably and refer to absorbent articles which are typically applied to the wearer by first leading the feet into the respective leg openings and subsequently pulling the pants from the feet to waist area over the hips and buttocks of the wearer and which are capable of being pulled up or down over the hips of the wearer. Typically, such articles may include a front waist portion and a back waist portion which may be connected about the hips of the wearer by integral or releasable members. A pant may be preformed by any suitable technique including, but not limited to, joining together portions of the article using refastenable and/or nonrefastenable bonds (e.g., seam, weld, adhesive, cohesive bond, fastener, etc.). A pant may be preformed anywhere along the circumference of the article (e.g., side fastened, front waist fastened).

"Polymer" as used herein refers to but is not limited to, homopolymers, copolymers, such as for example, block, graft, random and alternating copolymers, terpolymers, etc. and blends and modifications thereof. Unless otherwise specifically limited, the term "polymer" includes all possible spatial configurations of the molecule and include, but are not limited to isotactic, syndiotactic and random symmetries.

"Rear" as used herein refers to the portion of an absorbent article or part thereof that is intended to be positioned proximate the back of the wearer.

"Release structure", "release region", "release surface" or "release material" and the like as used herein are used interchangeably and refer to a structure in fluid communication with the absorbent core having a larger relative liquid absorption capacity and/or rate allowing it to quickly take up, temporarily hold and releasing liquids.

"Resin" as used herein refers to a solid or semisolid polymeric material.

"Thermobonding" as used herein refers to a method of bonding fibers by the use of heat and/or high-pressure.

"Thermoplastic" as used herein refers to polymeric materials that have a melting temperature and can flow or be formed into desired shapes on the application of heat at or below the melting point.

"Ultrasonic" as used herein refers to the use of high frequency sound to generate localized heat through vibration thereby causing thermoplastic fibers to bond to one another.

"Water-absorbing", "liquid-absorbing", "absorbent", "absorbing" and the like as used herein are used interchangeably and refer to compounds, materials, products that absorb at least water, but typically also other aqueous fluids and typically other parts of bodily exudates such as at least urine or blood.

"Wearer facing layer" as used herein refers to elements of the chassis that form the inner surface of the absorbent article, such as the topsheet, the leg cuffs, and the side panels, etc., when such elements are present.

"Weaving" as used herein refers to the process of interlacing two or more sets of yarns at right angles to form a fabric; a web of fibers produced by weaving is herein referred to as a "woven".

"Web material" as used herein refers to an essentially endless material in one direction, i.e. the longitudinal extension or the length, or the x- direction in Cartesian coordinates relative to the web material. Included in this term is an essentially unlimited sequence of pieces cut or otherwise separated from an essentially endless material. Often, though not necessarily, the web materials will have a thickness dimension (i.e. the z-direction) which is significantly smaller than the longitudinal extension (i.e. in x-direction). Typically, the width of web materials (they-direction) will be significantly larger than the thickness, but less than the length. Often, though not necessarily, the thickness and the width of such materials is essentially constant along the length of the web. Without intending any limitation, such web materials may be cellulosic fiber materials, tissues, woven or nonwoven materials and the like. Typically, though not necessarily, web materials are supplied in roll form, or on spools, or in a folded state in boxes. The individual deliveries may then be spliced together to form the essentially endless structure. A web material may be composed of several web materials, such as multilayer non-woven, coated tissues, nonwoven/film laminates. Web materials may comprise other materials, such as added binding material, particles, hydrophilizing agents and the like.

"Wet burst strength" is a measure of a layer's ability to absorb energy, when wet and subjected to deformation normal to the plane of the web.

"Wet strength" as used herein refers to the strength of a joint determined immediately after removal from a liquid in which it has been immersed under specified conditions of time, temperature and pressure. The term is commonly used in the art to designate strength after immersion in water.

"Wetlaying" as used herein refers to the forming a web from an aqueous dispersion of fibers by applying modified paper making techniques; a web of fibers produced by wetlaying is herein referred to as a "wetlaid".

"Wood pulp" as used herein refers to cellulosic fibers used to make viscose rayon, paper and the absorbent cores of products such as feminine hygiene garments, baby diapers and pants and adult incontinence garments.

"X-y dimension" as used herein refers to the plane orthogonal to the thickness of the article, structure or element. The x- and y-dimensions correspond generally to the width and length, respectively, of the article, structure or element.

"Z-dimension" as used herein refers to the dimension orthogonal to the length and width of the article, structure or element. The z-dimension corresponds generally to the thickness of the article, structure or element.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

The same or similar features and components are indicated with the same reference numerals throughout the figures.

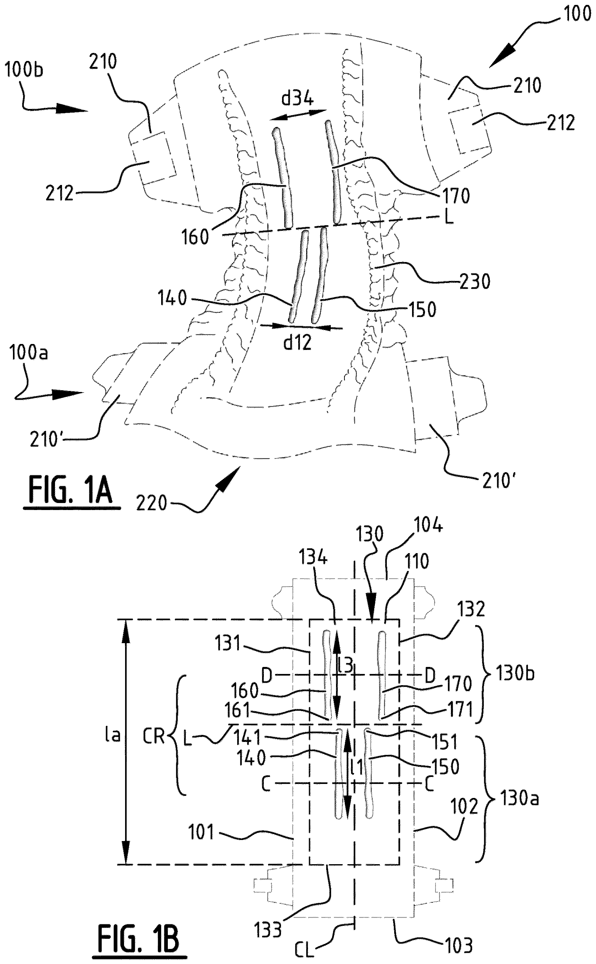

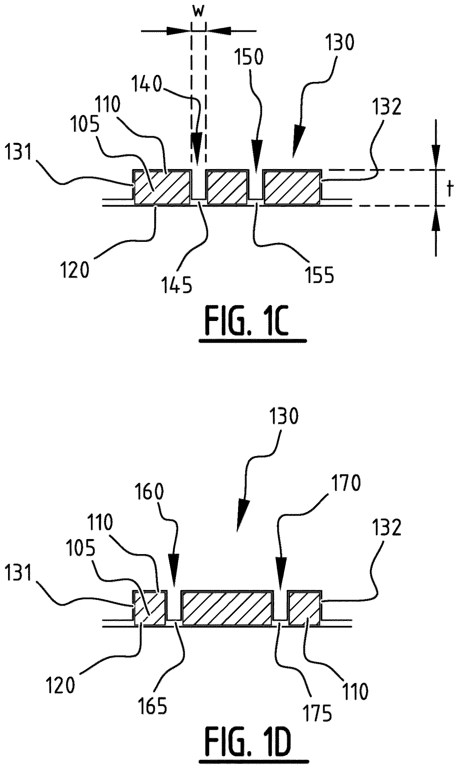

Figure 1A, 1B, 1C and 1D illustrate an exemplary embodiment of an absorbent article, here a diaper. Figure 1B shows the absorbent article in its flat out, un-contracted state with the wearer side facing the viewer. The skilled person understands that the absorbent article may also be a pant or an adult incontinence garment or the like. The absorbent article 100 comprises a liquid pervious topsheet, a liquid impervious backsheet, and an absorbent core 130 positioned in between the topsheet and the backsheet. The absorbent core 130 comprises absorbent material 105 between a top core wrap sheet 110 and a back core wrap sheet 120. Absorbent core 130 has a first and second longitudinal edge 131, 132 and a first and second transverse edge 133, 134.

The absorbent core 130 is provided with a plurality of attachment zones 145, 155, 165, 175 comprising at least a first attachment zone 145 and a second attachment zone 155. The first and second attachment zones extend next to each other from the crotch region CR in the direction of the first and/or second transverse edge 133, 134. In first and second attachment zone 145, 155 the top core wrap sheet 110 is attached to the back core wrap sheet 120
- along an attachment which extends, seen in a transverse direction of the absorbent core, over a transverse distance which is at least 1 mm, preferably at least 2 mm, more preferably at least 3mm, most preferably at least 4mm; and/or
- along a discontinuous attachment at a plurality of locations at a distance of each other, seen in the transverse direction of the absorbent core. In that manner, upon wetting of the absorbent material, a first and second channel 140, 150 are created at said first and second attachment zone 145, 155, respectively.

Absorbent article 100 is provided at said top core wrap sheet with at least a first and a second attachment zone 145, 155 located a distance d12 of each other. In that manner a first and second channel 140, 150 formed upon wetting, each extend from a crotch region CR in the direction of the first transverse edge 133. Preferably the distance d12 is between 10 mm and 50 mm, more preferably between 15 and 30 mm. Preferably, the length of the first and second channel is substantially the same, more preferably the length 11 of the first channel and the length 12 of the second channel is between 60 mm and 140 mm, more preferably between 75 mm and 125 mm. Preferably, the distance between the first attachment zone 145 and the first longitudinal side 131 is between 20 and 30 mm, and the distance between the second attachment zone 155 and the second longitudinal side 132 is between 20 and 30 mm. Preferably, the distance between the first/second attachment zone 145, 155 and the transverse edge 133 is between 50 and 125 mm, more preferably between 75 and 115 mm.

First channel 140 and second channel 150 are substantially parallel and run in the longitudinal direction of absorbent core 130. However, it is also possible for first and second channel 140, 150 to extend under a small angle with respect to the longitudinal direction of absorbent core 130, e.g. an angle between 5 and 10°. For example, first and second attachment zone 145, 155 (and hence first and second channel 140, 150) may be diverging slightly outwardly in the direction of first transverse edge 133. Preferably first channel 140 and second channel 150 are arranged symmetrically with respect to a longitudinal center line CL of absorbent core 130.

Absorbent article 100 is further provided with a third and a fourth channel 160, 170 located at a distance d34 of each other. Third and fourth channel 160, 170 each extend from crotch region CR in the direction of second transverse edge 134. The distance d12 between first and second channel 140, 150 is different from the distance d34 between third and fourth channel 160, 170. Preferably the distance d34 is between 25 mm and 80 mm, more preferably between 35 mm and 55 mm. Preferably, the length of the third and fourth channel 160, 170 is substantially the same, more preferably the length 13 of the third channel and the length l4 of the fourth channel is between 30 mm and 130 mm, more preferably between 30 mm and 70 mm. Preferably, the distance between the third attachment zone 165/third channel 160 and the first longitudinal side 131 is between 20 and 30 mm, and the distance between the fourth attachment zone 175 and the second longitudinal side 132 is between 20 and 30 mm. Preferably, the distance between the third/fourth attachment zone 165, 175 and the transverse edge 134 is between 30 mm and 100 mm, more preferably between 40 mm and 75 mm.

Third channel 160 and fourth channel 170 are substantially parallel and run in the longitudinal direction of absorbent core 130. However, it is also possible for third and fourth channel 160, 170 to extend under a small angle with respect to the longitudinal direction of absorbent core 130, e.g. an angle between 5 and 10°. For example, third and fourth channel 160, 170 may be diverging slightly outwardly in the direction of second transverse edge 134. Preferably third channel 160 and fourth channel 170 are arranged symmetrically with respect to a longitudinal center line CL of absorbent core 130.

Preferably, the distance between an end point 141 of first channel 140 and an end point 161 of third channel 160 is smaller than 25 mm, more preferably smaller than 20 mm. Similarly, preferably, the distance between an end point 151 of second channel 150 and an end point 171 of fourth channel 170 is smaller than 25 mm, more preferably smaller than 20 mm. More preferably, endpoints 141, 151, 161 and 171 are located on substantially the same transverse line L functioning as a fold line along which the diaper can be folded in two.

First, second, third and fourth channel 140, 150, 160, 170 each have a bottom which forms the attachment zone 145, 155, 165, 175, see figurer 1C and figure 1D. At bottom 145, 155, 165, 175 top core wrap sheet 110 is attached to back core wrap sheet 120. The width w of the bottom, seen in a transverse direction of absorbent core 130, is preferably larger than 2 mm, more preferably larger than 3 mm and even more preferable larger than 4 mm. To that end the attachment between top core wrap sheet 110 and the back core wrap sheet 120 may be an attachment extending over a transverse distance which is at least 2 mm, preferably at least 3 mm, more preferably at least 4 mm; and/or the attachment may be a discontinuous attachment in a plurality of locations at a distance of each other, seen in a transverse direction of absorbent core 130. Preferably the attachment at the bottom between the top core wrap sheet and the back core wrap sheet is realized by any one of the following or a combination thereof: pressure bonding, thermobonding, sonic bonding, chemical bonding, adhesive, mechanical bonding.

Outside of the plurality of channels 140, 150, 160, 170, absorbent core 130 has a maximum thickness t. Preferably, each channel 140, 150, 160, 170 extends through at least 90 % of the maximum thickness of absorbent core 130, more preferably through 100% of the thickness of absorbent core 130, such that, in the channel 140, 150, 160, 170, substantially no absorbent material is present that between top core wrap sheet 110 and back core wrap sheet 120. It is noted that the channel 140, 150, 160, 170 may be located below and/or above the attachment zones 145, 155, 165, 175, as will be explained in more detail below with reference to figure 14.

In a possible embodiment the attachment 145, 155, 165, 175 between top core wrap sheet 110 and back core wrap sheet 120, here at a bottom of each channel 140, 150, 160, 170, is a semi-permanent attachment configured to release after having been in contact with urine for a predetermined period of time, wherein said predetermined period of time is preferably smaller than 30s.

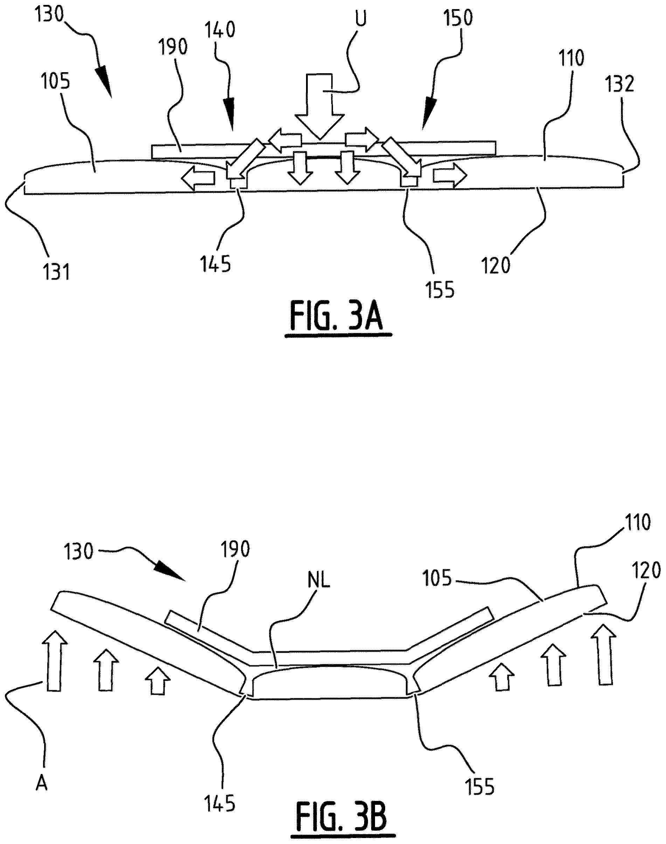

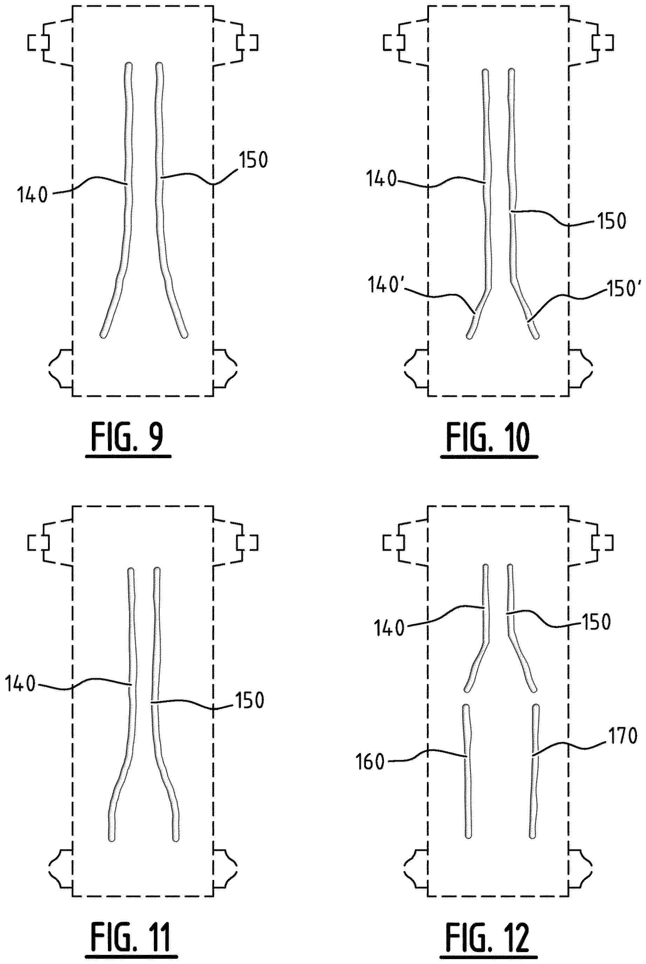

In another possible embodiment the attachment 145, 155, 165, 175 between top core wrap sheet 110 and back core wrap sheet 120, here at the bottom of each channel 140, 150, 160, 170, is a permanent attachment; and absorbent core 130 is configured such that, in a wetted state of absorbent core 130, the absorbent material extends over bottom 145, 155, 165, 175 of channel 140, 150, 160, 170. This is illustrated in figures 3A and 3B for first and second channels 140, 150. Channels 140, 150, 160, 170 guide urine U or any other aqueous liquid through the side walls of channels 140, 150, 160, 170 into absorbent core 130. Those side walls create an additional path along which the liquid can flow into absorbent core 130 and enhance the diffusion of the liquid into absorbent core 130. Also, because of the swelling of the core material of absorbent core 130, the outer bands of absorbent core 130 will rotate around channels 140, 150, 160, 170 as indicated by arrows A in figure 9B. In that manner the diaper takes the shape of a tub or cup, such that any liquid NL which would not yet be absorbed by the absorbent material 105 is maintained in the tub shape. This results in a better protection against leakage and a diaper fitting perfectly to the body. Hence the diaper of figures 1A-1D will create more freedom of movement for the wearer of a wetted diaper.

It is clear to the skilled person that the attachment zones may be provided by means of continuous attachments in the transversal direction of the absorbent core and/or continuous attachments in the longitudinal direction of the absorbent core and/or discontinuous attachments in the transversal direction of the absorbent core and/or discontinuous attachments in the longitudinal direction of the absorbent core.

Absorbent core 130 has a front portion 130a extending at one side of a transverse crotch line which corresponds in this embodiment with fold line L, and a rear portion 130b extending at the other side of the transverse crotch line L. First and second channel 140, 150 extend at least in front portion 130a of absorbent core 130, and third and fourth channel 160, 170 extend at least in rear portion 130b of the absorbent core 130. Preferably the distance d12 between first and second channel 140, 150 in front portion 130a is smaller than the distance d34 between third and fourth channel 160, 170 in rear portion 130b.

The plurality of channels 140, 150, 160, 170 together cover at least 60%, preferably at least 70% of the length la of absorbent core 130; indeed, in the embodiment of figure 1A-1D the channels cover a length equal to l1+l3 which is more than 60% of the length la of absorbent core 130.

The plurality of channels 140, 150, 160, 170 may be indicated with a color and/or with a pattern which is different from the color and/or pattern of topsheet. More in particular the area of the channels may comprise a print allowing a user to visually distinguish the channels. This print may be arranged on the topsheet, on the top core wrap sheet, on the back core wrap sheet, on the backsheet, or on any sheet in between the topsheet and the backsheet, as long as it is visible for a user. As the sheets may be partially transparent, the print may be arranged on a sheet in between the topsheet and the backsheet, as long as it is visible through the topsheet and/or the backsheet. Preferably the print is visible when looking at the topsheet of the diaper. For example, a topsheet area above first and second channels 140, 150 may be printed with an ink of a first color and a topsheet area above third and fourth channels 160, 170 may be printed with the same color or with a different color. In that manner a user will be able to easily recognize the front and rear portion of a diaper, and will recognize more easily how to put on the diaper.

The chassis of the diaper 100 in figures 1A-1D comprises a liquid pervious topsheet (not shown in figures 1C and 1D, but the topsheet is a layer above top core wrap sheet 110) and liquid impervious backsheet (not shown in figures 1C and 1D, but the backsheet is a layer below back core wrap sheet 110). The topsheet may be attached to the top core wrap sheet 110, e.g. in the attachment zones 140, 150, 160, 170. Also, the backsheet may be attached to the back core wrap sheet 120, e.g. in the attachment zones 140, 150, 160, 170. Preferably the chassis further includes side panels or ears 210, elasticized leg cuffs 230 and elastic waist elements (not shown). A front end portion of diaper 100 is configured as a front waist region 100a. The opposite rear end portion is configured as a back waist region 100b of diaper 100. An intermediate portion of diaper 100 is configured as crotch region CR, which extends longitudinally between first and second waist regions 100a and 100b. Waist regions 100a and 100b may include elastic waist elements such that they gather about the waist of the wearer to provide improved fit and containment. Crotch region CR is that portion of diaper 100 which, when the diaper 100 is worn, is generally positioned between the wearer's legs. The periphery of diaper 100 is defined by the outer edges of the diaper 100 in which longitudinal edges 101, 102 run generally parallel to a longitudinal axis of diaper 100 and transverse end edges 103, 104 run between the longitudinal edges 101, 102 generally parallel to a transverse axis of diaper 100. The chassis also comprises a fastening system, which may include at least one fastening or securing member 212 and at least one landing zone 220. The various components within diaper 100 may be bound, joined or secured by any method known in the art, for example by adhesives in uniform continuous layers, patterned layers or arrays of separate lines, spirals or spots. Top core wrap sheet, topsheet, back core wrap sheet, backsheet, absorbent material and other components may be assembled in a variety of well-known configurations and are well known in the art.

Backsheet covers absorbent core 130 and preferably extends beyond the absorbent core 130 toward longitudinal edges 101, 102 and end edges 103, 104 of diaper 100 and may be joined with top sheet. Backsheet prevents bodily exudates absorbed by the absorbent core 130 and contained within diaper 100 from soiling other external articles that may contact the wearer, such as bed sheets and undergarments. In preferred embodiments, backsheet is substantially impervious to bodily exudates and comprises a laminate of a nonwoven and a thin plastic film such as a thermoplastic film. Backsheet may comprise breathable materials that permit vapor to escape from diaper 100 while still preventing bodily exudates from passing through backsheet. It may be semi-rigid, non-elastic and can be made fully or partially elasticized and include backing.

The top sheet which is located above the top core wrap sheet 110, is preferably soft, exhibits good strikethroughs and has a reduced tendency to rewet from the liquid absorbent material. Top sheet may be semi-rigid and non-elastic, or may be fully or partially elasticized. Topsheet is intended to be placed in close proximity to the skin of the wearer when diaper 100 is worn. Topsheet permits bodily exudates to rapidly penetrate it so as to flow more quickly toward absorbent core 130 via a top surface thereof and via the plurality of channels 140, 150, 160, 170, preferably not allowing such bodily exudates to flow back through topsheet. Topsheet may be constructed from any one of a wide range of liquid and vapor permeable, preferably hydrophilic, materials. The upper and lower surface of topsheet may be treated differently. Topsheet may include e.g. a surfactant on the upper surface so as to facilitate liquid transfer there through, especially at a central zone or area of topsheet located over absorbent core 130, and/or a hydrophobic agent on the lower surface to minimize the liquid contained within absorbent core 130 from contact wetting topsheet thereby reducing rewet values. Topsheet may be coated with a substance having rash preventing or rash reducing properties. Preferably, topsheet covers substantially the entire wearer facing area of diaper 100, including substantially all of front waist region 100a, back waist region 100b, and crotch region CR. Optionally, side panels 210, 210' and/or waist feature layers of the inner region may be formed from the same single topsheet material. Alternatively, topsheet may be formed from multiple different materials which vary across of topsheet. Such a multiple piece design allows for creation of preferred properties and different zones of the topsheet.

Absorbent core 130 may comprise any absorbent material that is generally compressible, conformable, non-irritating to the wearer's skin, and capable of absorbing and retaining bodily exudates. Absorbent core 130 may comprise a wide variety of liquid absorbent materials commonly used in absorbent articles. Preferably, absorbent core 130 comprises fluff material, typically cellulosic fluff pulp. However, in other embodiments, absorbent core 130 may be substantially fluffless and comprise superabsorbent polymers. Also, absorbent core 130 may comprise a combination of cellulosic fluff pulp and superabsorbent polymers. Absorbent core 130 may be configured to extend substantially the full length and/or width of diaper 100. However, as in the embodiment of figures 1A-1D, preferably absorbent structure 130 is not coextensive with the entire diaper 100 and is limited to certain regions of diaper 100 including crotch region CR. In various embodiments, the absorbent core 300 extends to the edges of diaper 100 but the absorbent material is concentrated in the crotch region CR or another target zone of the diaper 100. In figures 1A-1D, absorbent core 130 is shown as having a substantially rectangular configuration, however, absorbent core 130 may be shaped differently, such as, elliptical, dogbane shaped, T-shaped or I-shaped. More in particular the width of the front portion 130a may be smaller than the width of the rear portion 130b of the absorbent core.

Examples of commonly occurring absorbent materials used for absorbent core 130 are cellulosic fluff pulp, tissue layers, highly absorbent polymers (so called superabsorbents), absorbent foam materials, absorbent nonwoven materials or the like. It is common to combine cellulosic fluff pulp with superabsorbent polymers in an absorbent core. Superabsorbent polymers are water-swellable, water-insoluble organic or inorganic materials capable of absorbing at least about 20 times its weight and in an aqueous solution containing 0.9 weight percent of sodium chloride.

Diaper 100 may also utilize a pair of containment walls or cuffs 230. Each cuff 230 is a longitudinally extending wall structure preferably positioned on each side of absorbent core 130 and spaced laterally from the center line CL. Preferably, cuffs 230 are attached, for example, by adhesive or sonic bonding to the lower structure. Preferably, cuffs 230 are equipped with elastic members. When released or otherwise allowed relaxing, the elastic members retract inwardly. When diaper 100 is worn, the elastic members function to contract cuffs 230 about the buttocks and the thighs of the wearer in a manner, which forms a seal between diaper 100, the buttocks and the thighs.

The waist regions 100a and 100b each comprise a central region and a pair of side panels or ears 210, 210' which typically comprise the outer lateral portions of the waist regions. These side panels 210, 210' may be unitary with the chassis or may be attached or joined thereto by any means know in the art. Preferably, the side panels 210 positioned in the back waist region 100b are flexible, extensible and/or elastic in at least the lateral direction. In another embodiment the side panels 210 are non-elastic, semi-rigid, rigid and/or stiff. In order to keep diaper 100 in place about the wearer, preferably at least a portion of the back waist region 100b is attached by fastening or securing members 212 to at least a portion of the front waist region 100a. The fastening or securing members 212 may be e.g. adhesive, mechanical fasteners, hook and loop features, conceivable strings and/or combinations thereof. The fastening or securing members 212 may also be co-adhesive such that they adhere to each other but not other materials. Preferably the materials making up the fastening or securing members 212 are flexible, extensible and/or elastic, allowing them to better conform to the shape and movements of the body and thus, to reduce the likelihood that the fastening system will irritate or injure the wearer's skin. Alternatively, the absorbent article may be pants and the like. In this configuration, the absorbent article may or may not have fastening members.

Diaper 100 may also employ additional layers, such as an acquisition layer and/or dispersion layer situated between topsheet and absorbent core 130, and/or coverstock layers, and/or other layers situated between absorbent core 130 and backsheet. An acquisition layer and/or dispersion layer serves to slow down the flow so that the liquid has adequate time to be absorbed by absorbent core 130. Figure 3A and 3B show an acquisition layer 190 above top core wrap layer 110.

Diaper 100 may also include such other features, components and elements as are known in the art including waistbands, waist cap features, elastics and the like to provide better fit, containment and aesthetic characteristics. These features may be assembled in a variety of well-known configurations and are well known in the art.

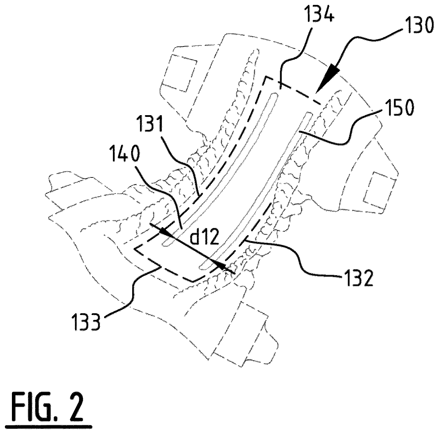

Figure 2 illustrates another more basic exemplary embodiment of a diaper 100. Diaper 100 comprises a liquid pervious topsheet, a liquid impervious backsheet, and an absorbent core 130 positioned in between topsheet and backsheet. Absorbent core 130 has a first and second longitudinal edge 131, 132 and a first and second transverse edge 133, 134. Absorbent article 100 is provided with a first and a second attachment zone for creating a first and a second channel 140, 150 located a distance d12 of each other, upon wetting of the diaper 100. First and second channel 140, 150 each extend from a crotch region CR in the direction of the first transverse edge 133 and the second transverse edge 134. In this embodiment, preferably, first and second channel extend over more than 80% of the length of absorbent core 130. Preferably the distance d12 is between 10 mm and 90 mm, more preferably between 20 mm and 80 mm, even more preferably between 30 mm and 50 mm. Preferably, the length of the first and second channel is substantially the same, more preferably the length 11 of the first channel and the length l2 of the second channel is between 100 mm and 350 mm, more preferably between 150 mm and 300 mm. Preferably, the distance between the first channel 140 and the first longitudinal side 131 is between 10 mm and 30 mm, and the distance between the second channel 150 and the second longitudinal side 132 is between 10 mm and 30 mm. Preferably, the distance between the first/second channel 140, 150 and the transverse edges 133, 134 is between 20 mm and 100 mm, more preferably between 30 mm and 75 mm.

First channel 140 and second channel 150 are substantially parallel and run in the longitudinal direction of absorbent core 130. However, it is also possible for first and second channel 140, 150 to extend under a small angle with respect to the longitudinal direction of absorbent core 130, e.g. an angle between 5 and 10°. For example, first and second channel 140, 150 may be diverging slightly outwardly in the direction of first transverse edge 133 and may be diverging slightly outwardly in the direction of second transverse edge 134. Preferably first channel 140 and second channel 150 are arranged symmetrically with respect to a longitudinal center line CL of absorbent core 130.

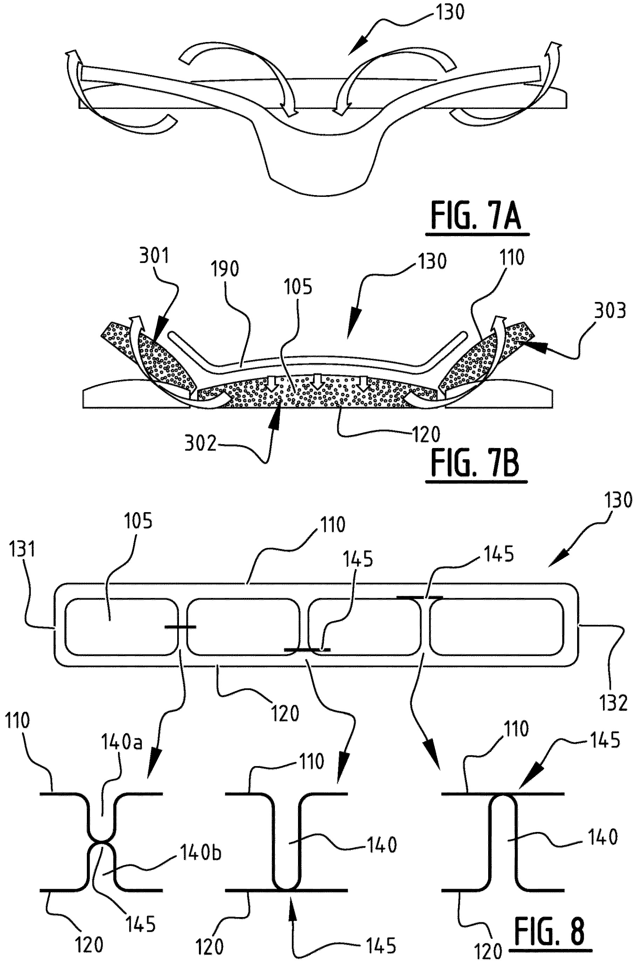

First and second channel 140, 150 may each have a bottom 145, 155, similar to the bottom illustrated in figurer 1C for the first embodiment of figures 1A-1D. However, it is noted that the channels 140, 150, 160, 170 may be located below and/or above the attachment zones 145, 155, 165, 175, as will be explained in more detail below with reference to figure 8.

At the attachment zones 145, 155, 165, 175 top core wrap sheet 110 is attached to back core wrap sheet 120 as described previously. Outside of the plurality of channels 140, 150, 160, 170 absorbent core 130 has a maximum thickness t. Preferably, in the unwetted state, each channel 140, 150, 160, 170 extends through at least 90 % of the maximum thickness of absorbent core 130, more preferably through 100% of the thickness of absorbent core 130, such that, in the channel 140, 150, 160, 170, substantially no absorbent material is present between top core wrap sheet 110 and back core wrap sheet 120.

The areas of the channels 140 and/or 150 and/or 160 and/or 170 may be indicated in a color and/or with a pattern which is different from the color and/or pattern of topsheet. More in particular the area of the channels may comprise a print allowing a user to visually distinguish the channels. This print may be arranged on the topsheet, on the top core wrap sheet, on the back core wrap sheet, on the backsheet, or on any sheet in between the topsheet and the backsheet, as long as it is visible for a user. Preferably the print is visible when looking at the topsheet of the diaper.

For example, a front portion of the channel 140 and/or 150 and/or 160 and/or 170 may be indicated with an ink of a first color and a rear portion the channels 140 and/or 150 and/or 160 and/or 170 may be indicated with a different color. In that manner a user will be able to easily recognize the front and rear portion of a diaper. Indeed, the user will know that the first color has to be on the left and the second color on the right. Hence he will recognize more easily how to put on the diaper. Topsheet, backsheet and absorbent core 130 may have the same features as described above in connection with figures 1A-1D.

It is clear to the skilled person that any embodiment described in view of baby diapers, is applicable in a similar manner to baby pants, mutatis mutandis.

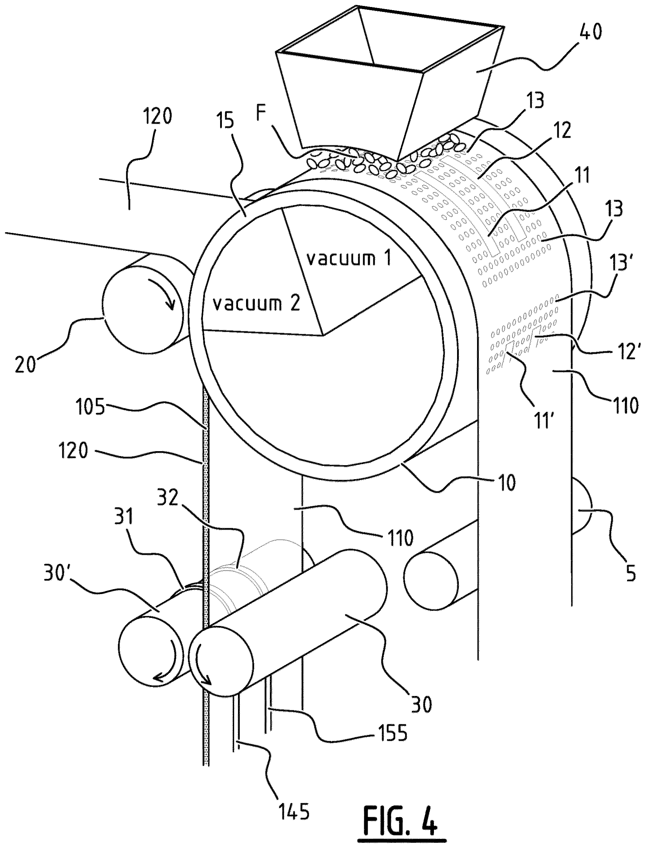

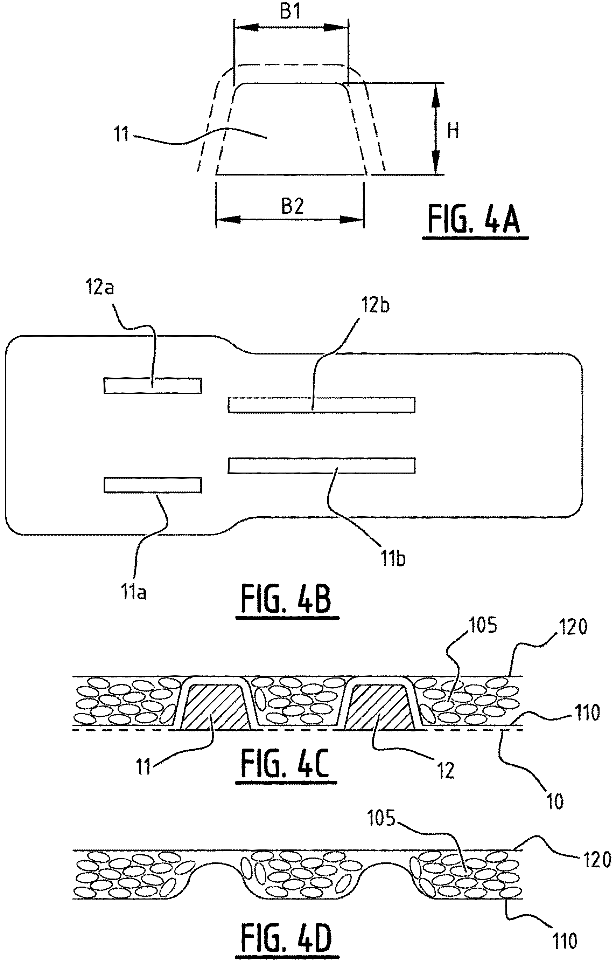

Figure 4 illustrates an embodiment of a method for manufacturing an absorbent article. The method comprises in a first step guiding a first sheet material 110 along an optional guide roller 5, and further along a rotating member 10, wherein a surface 15 of said rotating member 10 is provided with apattern with suction zones 13, 13' and non-suction zones 11, 12; 11', 12'. The illustrated configuration of non-suction zones 11, 12; 11', 12' does not fall within the scope of the claims. The first sheet material 110 is shown in a transparent manner to reveal the suction and non-suction zones of the rotating member 10. The suction zones 13, 13' may be provided with holes, and the non-suction zones 11, 12; 11', 12' are formed of closed material. For example, the non-suction zones 11, 12; 11', 12' may be provided with inserts as shown in figure 4A. As shown in figure 4A, the inserts 11, 12; 11', 12', may have a trapezoidal cross section. Figure 4B shows an insert pattern with four non-suction zones 11a, 11b, 12a, 12b per absorbent core. The illustrated configuration of non-suction zones 11a, 11b, 12a, 12b does not fall within the scope of the claims. The inserts may be fixed e.g. with screws on the rotating member 10. At an inner area of the rotating member 10 a vacuum is applied, see VACUUM 1. The non-suction zones 11, 12; 11', 12' comprise at least a first elongate zone 11, 11' and a second elongate zone 12, 12' extending in a circumferential direction of the rotating member 10. In a second step an absorbent material F is applied via a hopper 40 on said first sheet material 110 on the rotating member 10 such that the suction zones 13, 13' are covered with absorbent material and substantially no absorbent material is present on the non-suction zones 11, 12; 11', 12'. In a third step a second sheet material 120 is applied on top of the absorbent material on the first sheet material 110, e.g. using a further rotating member 20. This is shown also in figure 4C where a cross section through the absorbent core is shown during the application of the second sheet material 120. Figure 4D shows the cross section of the absorbent core downstream of rotating member 10. One of said first and second sheet material is a top core wrap sheet material, and the other one is a back core wrap sheet material. In the illustrated embodiment it is assumed that the first sheet material 110 is the top core wrap sheet material. In a fourth step the first sheet material 110 is attached to the second sheet material 120 at least in the areas where substantially no absorbent material is present, and such that at least a first and a second channel 140, 150 are formed in said top core wrap sheet material 110. The attaching may be done by applying pressure and heat on the top core wrap sheet material 110 and/or on the back core wrap sheet material 120 in the areas where substantially no absorbent material is present, e.g. by a rotating member 30 and/or opposite rotating member 30' which is provided with at least a first and a second seal rib 31, 32 dimensioned for applying pressure and heat on the top core wrap sheet material 110 in the areas where substantially no absorbent material is present in order to create the first and second channel 140, 150, respectively.

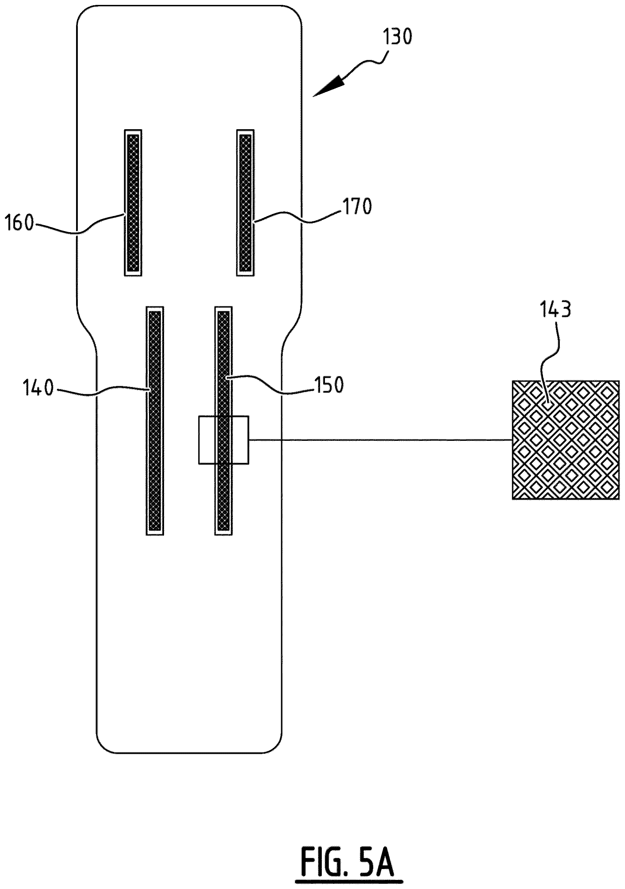

Figure 5A illustrates an exemplary embodiment of an absorbent core 130 with four attachment zones creating channels 140, 150, 160, 170. The illustrated configuration of channels 140, 150, 160, 170 does not fall within the scope of the claims. In the embodiment of figure 5A, the attachment zones are formed by welding the top core wrap sheet 110 to the back core wrap sheet 112. This welding may be done according to a predetermined sealing pattern. In the embodiment of figure 5A, the pattern consists of a plurality of discrete shapes 143, here a plurality of squares. Preferably, the discrete shapes 143 have dimensions smaller than 2 mm. Preferably, the distance between adjacent discrete shapes is between 0.5 and 3 mm.

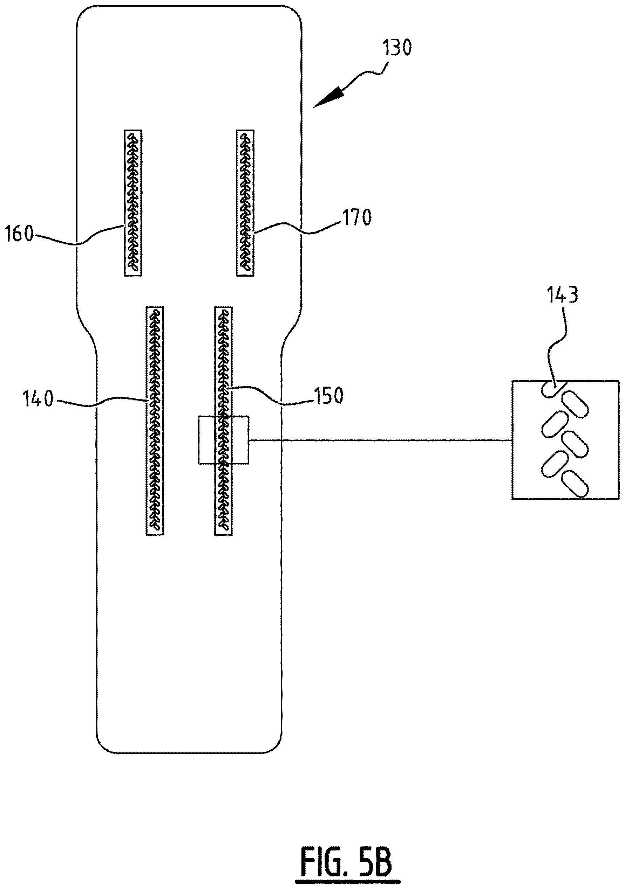

Figure 5B illustrates another exemplary embodiment of a sealing pattern that may be used in an embodiment of the invention. Here the pattern consists of a plurality of discrete shapes in the form of rounded elements 143. The rounded elements may have a length dimension between 0.5 mm and 5 mm, and a width dimension between 0.5 mm and 5 mm. Preferably, the discrete shapes are equally distributed in the attachment zones. The illustrated configuration of channels 140, 150, 160, 170 does not fall within the scope of the claims.

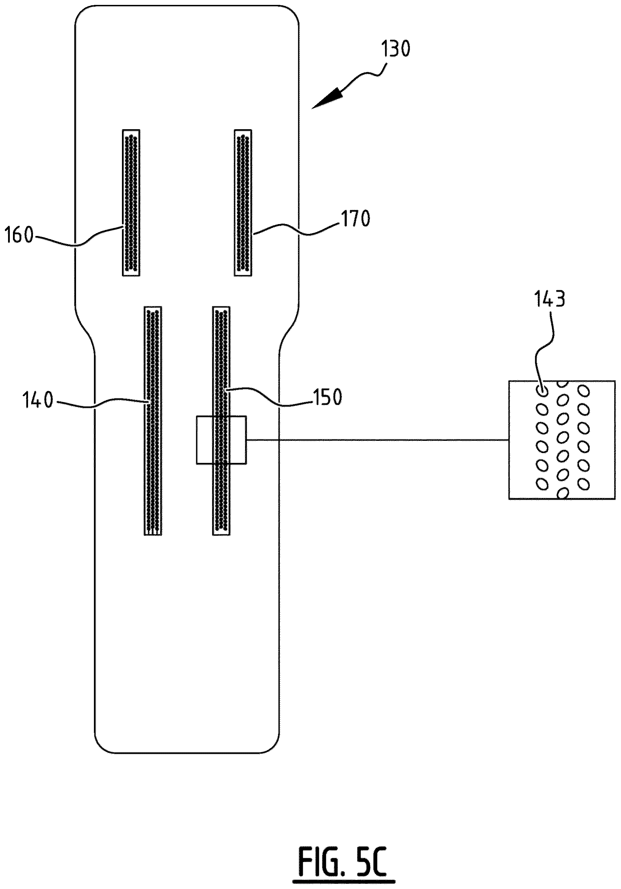

Figure 5C illustrates yet another embodiment where the sealing pattern consists of discrete shapes which are rounded. In this embodiment, three columns of rounded discrete elements 143 are used for each attachment zone 140, 150, 160, 170. The illustrated configuration of channels 140, 150, 160, 170 does not fall within the scope of the claims.

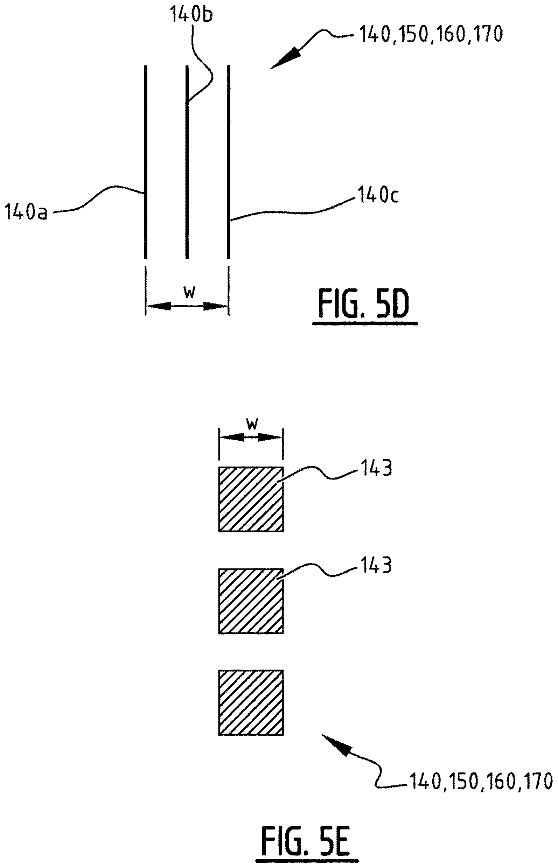

Figure 5D illustrates another exemplary embodiment of an attachment zone for creating a channel 140, 150, 160, 170. In this embodiment, the attachment zone is formed by a plurality of continuous line-shaped attachments 140a, 140b, 140c. The number of lines used may vary, and may be e.g. two lines or more than three adjacent lines. Preferably, the distance w between a first line 140a and a last line 140c is at least 1 mm, more preferably at least 2 mm, even more preferably more than 4 mm.

In the exemplary embodiment of figure 5E, the attachment zones creating channels 140, 150, 160, 170 may be formed of a plurality of discrete elements 143, wherein each discrete element has a width w which covers the entire width w of the attachment zone.

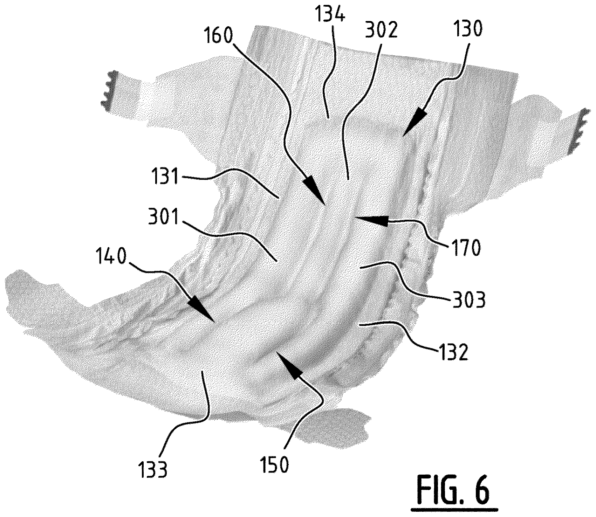

Figure 7A illustrates an exemplary embodiment of a traditional absorbent core. When a traditional absorbent core absorbs liquid, the core becomes bulky such that the diaper is no longer well adapted to the body. The liquid does not spread evenly but remains in the center of the absorbent core. Figure 7B illustrates an exemplary embodiment of an absorbent core of the invention. Thanks to the attachment zones and associated channels 140, 150, 160, 170, the liquid is evenly spread, resulting in the formation of tubes 301, 302, 303 which provide a tub shape to the absorbent core 130. Such a tub shape adapts perfectly to the body. Further, compared to prior art solutions, the liquid is kept in an improved manner absorbed in the absorbent core 130, and the risk on leakage is reduced. Also, because of the creation of the channels 140, 150, 160, 170, the liquid is absorbed faster. Figure 6 shows a perspective view of a diaper in the wetted state. Figure 6 clearly illustrates the formation of three tubes 301, 302, 303 giving the diaper a tub shape which is well adapted to the body.

Figure 8 illustrates an absorbent core 130 comprising an absorbent material 105 between a top core wrap sheet 110 and a back core wrap sheet 120. The absorbent core has a first and second longitudinal edge 131, 132. The absorbent core 130 is provided with a plurality of attachment zones 145. Figure 8 illustrates that the attachment zones 145 may be positioned at different locations. As illustrated on the left in figure 8, the attachment zone may be positioned more or less centrally such that an upper channel portion 140a and a lower channel portion 140b is formed. In an alternative embodiment, the attachment zone 145 may be positioned at the bottom such that an upper channel 140 is created, see the example in the middle of figure 8. According to yet another embodiment, the attachment zone 145 may be located at the top, such that the channel 140 is formed below top core wrap sheet 110. The skilled person understands that any variants thereof are also possible, as long as the attachment zones allow the formation of channels upon wetting of the absorbent core 130.

Although the method is illustrated for two channels, the skilled person understands that the method can be adapted for forming three, four or more channels, and in particular for manufacturing any one of the absorbent articles disclosed in the present application.

Figures 9-14 illustrate multiple advantageous positions for the attachment zones in an absorbent core according to the invention.

According to the exemplary embodiment of figure 9 the plurality of attachment zones comprises a first attachment zone 140 and a second attachment zone 150. The first and second attachment zones 140 are substantially parallel in the crotch region and diverge in the direction of a front transverse edge of absorbent core.

According to the exemplary embodiment of figure 10 the plurality of attachment zones comprises a first longitudinal attachment zone 140 and a second longitudinal attachment zone 150, wherein front end portions 140', 150' thereof diverge in the direction of the front transverse edge of the absorbent core.

According to the exemplary embodiment of figure 11 the plurality of attachment zones comprises a first attachment zone 140 and a second attachment zone 150. The first and second attachment zones 140 are substantially parallel in a rear part of the crotch region, whilst the transverse distance between the first and second attachment zones gradually increases in the direction of a front transverse edge of absorbent core.

Figure 12 illustrates one or more longitudinal sections 140, 150, 160, 170 and/or one or more inclined sections.

Whilst the principles of the invention have been set out above in connection with specific embodiments, it is to be understood that this description is merely made by way of example and not as a limitation of the scope of protection which is determined by the appended claims.

## Claims

1. An absorbent article (100) comprising a liquid pervious topsheet, a liquid impervious backsheet, and an absorbent core (130) comprising an absorbent material between a top core wrap sheet and a back core wrap sheet, said absorbent core (130) being positioned in between said topsheet and said backsheet, said absorbent core (130) having a first and second longitudinal edge and a first and second transverse edge; wherein the absorbent core (130) is provided with a plurality of attachment zones comprising at least a first and a second attachment zone (145, 155) located at a distance of each other, said first and second attachment zone (145, 155) extending next to each other from a crotch region in the direction of the first and/or second transverse edge; wherein in said first and second attachment zone:
said top core wrap sheet is attached to said back core wrap sheet along an attachment which extends, seen in a transverse direction of the absorbent core (130), over a transverse distance which is at least 1 mm, preferably at least 2 mm, more preferably at least 3mm, most preferably at least 4 mm; and/or
said top core wrap sheet is attached to said back core wrap sheet along a discontinuous attachment at a plurality of locations at a distance of each other, seen in the transverse direction of the absorbent core (130);
such that upon wetting of the absorbent material, a first and second channel (140, 150) are created at said first and second attachment zone (145, 155), respectively,
wherein the first and second attachment zones (145, 155) are parallel in a rear part of the crotch region, whilst the transverse distance between the first and second attachment zones (145, 155) gradually increases in the direction of the first transverse edge of the absorbent core (130), wherein the first transverse edge is a front transverse edge of the absorbent core (130).

2. The absorbent article (100) of claim 1, wherein the first attachment zone (145) and the second attachment zone (155) extend in a longitudinal direction of the absorbent core (130).

3. The absorbent article (100) of any one of the previous claims, wherein the attachment between the top core wrap sheet and the back core wrap sheet in the first and the second attachment zone (145, 155) is a permanent attachment such that, in a wetted state of the absorbent material, the absorbent material extends over the first and second attachment zone (145, 155).

4. The absorbent article (100) of any one of the previous claims, wherein the plurality of attachment zones together cover at least 30 %, preferably at least 40%, preferably at least 50%, more preferably at least 60%, more preferably at least 70%, more preferably 80% and more preferably at least 90%of a length of the absorbent core (130).

5. The absorbent article (100) of any one of the previous claims, wherein, outside of the plurality of attachment zones the absorbent core (130) has a maximum thickness; wherein the first and second attachment zone (145, 155) extend through at least 90 % of the maximum thickness of the absorbent core (130), more preferably through 100% of the thickness of the absorbent core (130) such that in the first and second attachment zone (145, 155) substantially no absorbent material is present between the top core wrap sheet and the back core wrap sheet.

6. The absorbent article (100) of any one of the previous claims, wherein the first attachment zone (145) and the second attachment zone (145) are arranged symmetrically with respect to a longitudinal center line of the absorbent core (130) extending between the first and second transverse edge.

7. The absorbent article (100) of any one of the previous claims, wherein the attachment between the top core wrap sheet and the back core wrap sheet is any one of the following or a combination thereof: pressure bonding, thermal bonding, sonic bonding, chemical bonding, adhesive.

8. The absorbent article (100) of any one of the previous claims, wherein the plurality of attachment zones further comprises a third and a fourth attachment zone (160, 170) located at a distance of each other, said third and fourth attachment zone each extending in the direction of the first and/or second transverse edge.

9. The absorbent article (100) of claim 8, wherein the absorbent core (130) has a front portion extending at one side of a transverse crotch line and a rear portion extending at the other side of the transverse crotch line; wherein the first and second attachment zone extend at least in the front portion of the absorbent core (130); and wherein the third and fourth attachment zone extend at least in the rear portion of the absorbent core (130).

10. The absorbent article (100) of any one of the claims 8-9, wherein the first attachment zone is connected to the third attachment zone through a first transverse attachment zone and wherein the second attachment zone is connected to the fourth attachment zone through a second transverse attachment zone.

11. The absorbent article (100) of claim 8, wherein the first and the second attachment zone extend in a longitudinal direction of the absorbent core (130) over a length which is longer than the length of the third and fourth attachment zone, wherein the first and the second attachment zone are located between the third and fourth attachment zone.

12. The absorbent article (100) of any one of the claims 8-11, wherein the third attachment zone and the fourth attachment zone are arranged symmetrically with respect to a longitudinal center line of the absorbent core (130) extending between the first and second transverse edge.

13. The absorbent article (100) of any one of the previous claims, wherein the distance between the first and the second attachment zone is between 10 mm and 50 mm, preferably between 15 mm and 30 mm.

14. The absorbent article (100) of any one of the previous claims, wherein the length of the first and the second attachment zone is larger than 60 mm, preferably larger than 70 mm.

15. The absorbent article (100) of any one of the previous claims, wherein the absorbent material comprises cellulosic fluff pulp.

16. The absorbent article (100) of any one of the previous claims, wherein the absorbent material contains less than 20% by weight fluff pulp.

17. The absorbent article (100) of any one of the previous claims, wherein substantially no absorbent material is present in the first and second attachment zone.

18. The absorbent article (100) of any one of the previous claims, wherein the first and second attachment zone each have a bottom and a top, wherein the top core wrap sheet is attached to the back core wrap sheet at said bottom, at said top, or between said bottom and said top.

19. A method for manufacturing an absorbent article as defined in claims 1-18, said method comprising:
a. guiding a first sheet material (110) along a rotating member (10), wherein a surface (15) of said rotating member is provided with a pattern with suction zones (13, 13') and non-suction zones (11, 12; 11', 12'); wherein said non-suction zones comprise at least a first and a second elongate zone extending in a circumferential direction of the rotating member;
b. applying an absorbent material on said first sheet material on the rotating member such that the suction zones are covered with absorbent material and substantially no absorbent material is present on the non-suction zones;
c. applying a second sheet material (120) on top of the absorbent material on the first sheet material; wherein one of said first and second sheet material is a top core wrap sheet material, and the other one is a back core wrap sheet material;
d. attaching said first sheet material (110) to said second sheet material (120) at least in the areas where substantially no absorbent material is present, and such that at least a first and a second attachment zone (140, 150) are formed located at a distance of each other,
wherein the first and second attachment zones are parallel in a rear part of the crotch region, whilst the transverse distance between the first and second attachment zones gradually increases in the direction of the first transverse edge of the absorbent core (130), wherein the first transverse edge is a front transverse edge of the absorbent core (130).

20. The method of claim 19, wherein the attaching is done by applying pressure and heat on the top core wrap sheet material (110) and/or the back core wrap sheet material in the areas where substantially no absorbent material is present.

21. The method of claim 20 wherein the attaching is done by a rotating member (30) which is provided with at least a first and a second seal rib (31, 32) dimensioned for applying pressure and heat on the top core wrap sheet material (110) and/or the back core wrap sheet material in the areas where substantially no absorbent material is present in order to create the first and second attachment zone (140, 150), respectively.

## Patentansprüche

1. Absorptionsartikel (100), umfassend eine flüssigkeitsdurchlässige Oberschicht, eine flüssigkeitsundurchlässige Unterschicht und einen Absorptionskern (130), der ein Absorptionsmaterial zwischen einer oberen Kernhüllschicht und einer unteren Kernhüllschicht umfasst, wobei der Absorptionskern (130) zwischen der Oberschicht und der Unterschicht positioniert ist, wobei der Absorptionskern (130) eine erste und eine zweite Längskante und eine erste und eine zweite Querkante aufweist; wobei der Absorptionskern (130) mit einer Vielzahl von Befestigungszonen versehen ist, die mindestens eine erste und eine zweite Befestigungszone (145, 155) umfasst, die sich in einem Abstand voneinander befinden, wobei sich die erste und zweite Befestigungszone (145, 155) nebeneinander von einer Schrittregion in Richtung der ersten und/oder zweiten Querkante erstrecken; wobei in der ersten und zweiten Befestigungszone:
die obere Kernhüllschicht an der unteren Kernhüllschicht entlang einer Befestigung befestigt ist, die sich in Querrichtung des Absorptionskerns (130) über einen Querabstand erstreckt, der mindestens 1 mm, vorzugsweise mindestens 2 mm, mehr bevorzugt mindestens 3 mm, am meisten bevorzugt mindestens 4 mm beträgt; und/oder
die obere Kernhüllschicht an der unteren Kernhüllschicht entlang einer diskontinuierlichen Befestigung an einer Vielzahl von Stellen befestigt ist, die sich in Querrichtung des Absorptionskerns (130) gesehen in einem Abstand voneinander befinden;
sodass beim Benetzen des Absorptionsmaterials ein erster und zweiter Kanal (140, 150), jeweils an der ersten und zweiten Befestigungszone (145, 155), erzeugt werden,
wobei die erste und zweite Befestigungszone (145, 155) in einem hinteren Teil der Schrittregion parallel sind, während der Querabstand zwischen der ersten und zweiten Befestigungszone (145, 155) in Richtung der ersten Querkante des Absorptionskerns (130) allmählich zunimmt, wobei die erste Querkante eine vordere Querkante des Absorptionskerns (130) ist.

2. Absorptionsartikel (100) nach Anspruch 1, wobei sich die erste Befestigungszone (145) und die zweite Befestigungszone (155) in einer Längsrichtung des Absorptionskerns (130) erstrecken.

3. Absorptionsartikel (100) nach einem der vorstehenden Ansprüche, wobei die Befestigung zwischen der oberen Kernhüllschicht und der unteren Kernhüllschicht in der ersten und der zweiten Befestigungszone (145, 155) eine permanente Befestigung ist, sodass sich das Absorptionsmaterial in einem benetzten Zustand des Absorptionsmaterials über die erste und zweite Befestigungszone (145, 155) erstreckt.

4. Absorptionsartikel (100) nach einem der vorstehenden Ansprüche, wobei die Vielzahl von Befestigungszonen zusammen mindestens 30 %, vorzugsweise mindestens 40 %, vorzugsweise mindestens 50 %, mehr bevorzugt mindestens 60 %, mehr bevorzugt mindestens 70 %, mehr bevorzugt 80 % und mehr bevorzugt mindestens 90 % einer Länge des Absorptionskerns (130) bedecken.

5. Absorptionsartikel (100) nach einem der vorstehenden Ansprüche, wobei der Absorptionskern (130) außerhalb der Vielzahl von Befestigungszonen eine maximale Dicke aufweist; wobei sich die erste und zweite Befestigungszone (145, 155) durch mindestens 90 % der maximalen Dicke des Absorptionskerns (130), mehr bevorzugt durch 100 % der Dicke des Absorptionskerns (130) hindurch erstrecken, sodass in der ersten und zweiten Befestigungszone (145, 155) im Wesentlichen kein Absorptionsmaterial zwischen der oberen Kernhüllschicht und der unteren Kernhüllschicht vorhanden ist.

6. Absorptionsartikel (100) nach einem der vorstehenden Ansprüche, wobei die erste Befestigungszone (145) und die zweite Befestigungszone (145) in Bezug auf eine Längsmittellinie des Absorptionskerns (130), der sich zwischen der ersten und der zweiten Querkante erstreckt, symmetrisch angeordnet sind.

7. Absorptionsartikel (100) nach einem der vorstehenden Ansprüche, wobei die Befestigung zwischen der oberen Kernhüllschicht und der unteren Kernhüllschicht eine beliebige der Folgenden oder eine Kombination davon ist: Druckbindung, Wärmebindung, Schallbindung, chemische Bindung, Klebstoff.

8. Absorptionsartikel (100) nach einem der vorstehenden Ansprüche, wobei die Vielzahl von Befestigungszonen ferner eine dritte und eine vierte Befestigungszone (160, 170) umfasst, die sich in einem Abstand voneinander befinden, wobei sich die dritte und vierte Befestigungszone jeweils in Richtung der ersten und/oder zweiten Querkante erstrecken.

9. Absorptionsartikel (100) nach Anspruch 8, wobei der Absorptionskern (130) einen vorderen Abschnitt, der sich auf einer Seite einer querverlaufenden Schrittlinie erstreckt, und einen hinteren Abschnitt aufweist, der sich auf der anderen Seite der querverlaufenden Schrittlinie erstreckt; wobei sich die erste und zweite Befestigungszone mindestens in dem vorderen Abschnitt des Absorptionskerns (130) erstrecken; und wobei sich die dritte und vierte Befestigungszone mindestens in dem hinteren Abschnitt des Absorptionskerns (130) erstrecken.

10. Absorptionsartikel (100) nach einem der Ansprüche 8 bis 9, wobei die erste Befestigungszone durch eine erste querverlaufende Befestigungszone mit der dritten Befestigungszone verbunden ist und wobei die zweite Befestigungszone durch eine zweite querverlaufende Befestigungszone mit der vierten Befestigungszone verbunden ist.

11. Absorptionsartikel (100) nach Anspruch 8, wobei sich die erste und die zweite Befestigungszone in einer Längsrichtung des Absorptionskerns (130) über eine Länge erstrecken, die länger ist als die Länge der dritten und vierten Befestigungszone, wobei sich die erste und die zweite Befestigungszone zwischen der dritten und vierten Befestigungszone befinden.

12. Absorptionsartikel (100) nach einem der Ansprüche 8 bis 11, wobei die dritte Befestigungszone und die vierte Befestigungszone in Bezug auf eine Längsmittellinie des Absorptionskerns (130), der sich zwischen der ersten und der zweiten Querkante erstreckt, symmetrisch angeordnet sind.

13. Absorptionsartikel (100) nach einem der vorstehenden Ansprüche, wobei der Abstand zwischen der ersten und der zweiten Befestigungszone zwischen 10 mm und 50 mm, vorzugsweise zwischen 15 mm und 30 mm, beträgt.

14. Absorptionsartikel (100) nach einem der vorstehenden Ansprüche, wobei die Länge der ersten und der zweiten Befestigungszone größer als 60 mm, vorzugsweise größer als 70 mm ist.

15. Absorptionsartikel (100) nach einem der vorstehenden Ansprüche, wobei das Absorptionsmaterial cellulosischen Flockenzellstoff umfasst.

16. Absorptionsartikel (100) nach einem der vorstehenden Ansprüche, wobei das Absorptionsmaterial weniger als 20 Gewichts-% Flockenzellstoff enthält.

17. Absorptionsartikel (100) nach einem der vorstehenden Ansprüche, wobei im Wesentlichen kein Absorptionsmaterial in der ersten und zweiten Befestigungszone vorhanden ist.

18. Absorptionsartikel (100) nach einem der vorstehenden Ansprüche, wobei die erste und zweite Befestigungszone jeweils eine Unterseite und eine Oberseite aufweisen, wobei die obere Kernhüllschicht an der Unterseite, an der Oberseite oder zwischen der Unterseite und der Oberseite an der unteren Kernhüllschicht befestigt ist.

19. Verfahren zum Herstellen eines Absorptionsartikels wie in Ansprüchen 1 bis 18 definiert, wobei das Verfahren umfasst:
a. Führen eines ersten Schichtmaterials (110) entlang eines rotierenden Elements (10), wobei eine Oberfläche (15) des rotierenden Elements mit einem Muster mit Saugzonen (13, 13') und Nicht-Saugzonen (11, 12; 11', 12') versehen ist; wobei die Nicht-Saugzonen mindestens eine erste und eine zweite längliche Zone umfassen, die sich in einer Umfangsrichtung des rotierenden Elements erstrecken;
b. Aufbringen eines Absorptionsmaterials auf das erste Schichtmaterial auf dem rotierenden Element, sodass die Saugzonen mit Absorptionsmaterial bedeckt sind und in den Nicht-Saugzonen im Wesentlichen kein Absorptionsmaterial vorhanden ist;
c. Aufbringen eines zweiten Schichtmaterials (120) auf das Absorptionsmaterial auf dem ersten Schichtmaterial; wobei eines von dem ersten und zweiten Schichtmaterial ein oberes Kernhüllschichtmaterial ist und das andere ein unteres Kernhüllschichtmaterial ist;
d. Aufbringen des ersten Schichtmaterials (110) auf das zweite Schichtmaterial (120) mindestens in den Bereichen, in denen im Wesentlichen kein Absorptionsmaterial vorhanden ist, und so, dass mindestens eine erste und eine zweite Befestigungszone (140, 150) gebildet werden, die sich in einem Abstand voneinander befinden,
wobei die erste und zweite Befestigungszone in einem hinteren Teil der Schrittregion parallel sind, während der Querabstand zwischen der ersten und zweiten Befestigungszone in Richtung der ersten Querkante des Absorptionskerns (130) allmählich zunimmt, wobei die erste Querkante eine vordere Querkante des Absorptionskerns (130) ist.

20. Verfahren nach Anspruch 19, wobei das Befestigen durch Aufbringen von Druck und Wärme auf das obere Kernhüllschichtmaterial (110) und/oder das untere Kernhüllschichtmaterial in den Bereichen erfolgt, in denen im Wesentlichen kein Absorptionsmaterial vorhanden ist.

21. Verfahren nach Anspruch 20, wobei das Befestigen durch ein rotierendes Element (30) erfolgt, das mit mindestens einer ersten und einer zweiten Dichtungsrippe (31, 32) versehen ist, die bemessen sind, um Druck und Wärme auf das obere Kernhüllschichtmaterial (110) und/oder das untere Kernhüllschichtmaterial in den Bereichen aufzubringen, in denen im Wesentlichen kein Absorptionsmaterial vorhanden ist, um jeweils die erste und zweite Befestigungszone (140, 150) zu erzeugen.

## Revendications

1. Article absorbant (100) comprenant une feuille supérieure perméable aux liquides, une feuille inférieure imperméable aux liquides et une âme absorbante (130) comprenant un matériau absorbant entre une feuille d'enveloppe centrale supérieure et une feuille d'enveloppe centrale inférieure, ladite âme absorbante (130) étant positionnée entre ladite feuille supérieure et ladite feuille inférieure, ladite âme absorbante (130) présentant un premier et un second bord longitudinal et un premier et un second bord transversal ; dans lequel l'âme absorbante (130) est pourvue d'une pluralité de zones de fixation comprenant au moins une première et une deuxième zones de fixation (145, 155) situées à une certaine distance l'une de l'autre, lesdites première et deuxième zones de fixation (145, 155) s'étendant l'une à côté de l'autre à partir d'une région d'entrejambe dans la direction du premier et/ou du second bord transversal ; dans lequel dans lesdites première et deuxième zones de fixation :
ladite feuille d'enveloppe centrale supérieure est fixée à ladite feuille d'enveloppe centrale inférieure le long d'une fixation qui s'étend, vue dans la direction transversale de l'âme absorbante (130), sur une distance transversale d'au moins 1 mm, préférablement d'au moins 2 mm, plus préférablement d'au moins 3 mm, le plus préférablement d'au moins 4 mm ; et/ou
ladite feuille d'enveloppe centrale supérieure est fixée à ladite feuille d'enveloppe centrale inférieure le long d'une fixation discontinue en une pluralité d'endroits à une certaine distance les uns des autres, vus dans la direction transversale de l'âme absorbante (130) ;
de telle sorte que lors du mouillage du matériau absorbant, un premier et un second canal (140, 150) sont créés au niveau desdites première et deuxième zones de fixation (145, 155), respectivement,
dans lequel les première et deuxième zones de fixation (145, 155) sont parallèles dans une partie arrière de la région d'entrejambe, tandis que la distance transversale entre les première et deuxième zones de fixation (145, 155) augmente progressivement en direction du premier bord transversal de l'âme absorbante (130), le premier bord transversal étant un bord transversal avant de l'âme absorbante (130).

2. Article absorbant (100) selon la revendication 1, dans lequel la première zone de fixation (145) et la deuxième zone de fixation (155) s'étendent dans une direction longitudinale de l'âme absorbante (130).

3. Article absorbant (100) selon l'une quelconque des revendications précédentes, dans lequel la fixation entre la feuille d'enveloppe centrale supérieure et la feuille d'enveloppe centrale inférieure dans les première et deuxième zones de fixation (145, 155) est une fixation permanente de telle sorte que, dans un état mouillé du matériau absorbant, le matériau absorbant s'étend sur les première et deuxième zones de fixation (145, 155).

4. Article absorbant (100) selon l'une quelconque des revendications précédentes, dans lequel la pluralité de zones de fixation couvrent ensemble au moins 30 %, préférablement au moins 40 %, préférablement au moins 50 %, plus préférablement au moins 60 %, plus préférablement au moins 70 %, plus préférablement 80 % et plus préférablement au moins 90 % d'une longueur de l'âme absorbante (130).

5. Article absorbant (100) selon l'une quelconque des revendications précédentes, dans lequel, en dehors de la pluralité de zones de fixation l'âme absorbante (130) présente une épaisseur maximale ; dans lequel les première et deuxième zones de fixation (145, 155) s'étendent sur au moins 90 % de l'épaisseur maximale de l'âme absorbante (130), plus préférablement sur 100 % de l'épaisseur de l'âme absorbante (130), de telle sorte que dans les première et deuxième zones de fixation (145, 155), il n'y a pratiquement pas de matériau absorbant entre la feuille d'enveloppe centrale supérieure et la feuille d'enveloppe centrale inférieure.

6. Article absorbant (100) selon l'une quelconque des revendications précédentes, dans lequel la première zone de fixation (145) et la deuxième zone de fixation (145) sont disposées symétriquement par rapport à une ligne centrale longitudinale de l'âme absorbante (130) s'étendant entre les premier et second bords transversaux.

7. Article absorbant (100) selon l'une quelconque des revendications précédentes, dans lequel la fixation entre la feuille d'enveloppe centrale supérieure et la feuille d'enveloppe centrale inférieure est l'une quelconque des éléments suivants ou une combinaison de ceux-ci : collage par pression, collage thermique, collage sonique, collage chimique, adhésif.

8. Article absorbant (100) selon l'une quelconque des revendications précédentes, dans lequel la pluralité de zones de fixation comprend en outre une troisième et une quatrième zone de fixation (160, 170) situées à une certaine distance l'une de l'autre, lesdites troisième et quatrième zones de fixation s'étendant chacune dans la direction du premier et/ou du second bord transversal.

9. Article absorbant (100) selon la revendication 8, dans lequel l'âme absorbante (130) présente une partie avant s'étendant d'un côté d'une ligne d'entrejambe transversale et une partie arrière s'étendant de l'autre côté de la ligne d'entrejambe transversale ; dans lequel les première et deuxième zones de fixation s'étendent au moins dans la partie avant de l'âme absorbante (130) ; et dans lequel les troisième et quatrième zones de fixation s'étendent au moins dans la partie arrière de l'âme absorbante (130).

10. Article absorbant (100) selon l'une quelconque des revendications 8 à 9, dans lequel la première zone de fixation est reliée à la troisième zone de fixation par l'intermédiaire d'une première zone de fixation transversale et dans lequel la deuxième zone de fixation est reliée à la quatrième zone de fixation par l'intermédiaire d'une deuxième zone de fixation transversale.

11. Article absorbant (100) selon la revendication 8, dans lequel les première et deuxième zones de fixation s'étendent dans la direction longitudinale de l'âme absorbante (130) sur une longueur supérieure à la longueur des troisième et quatrième zones de fixation, les première et deuxième zones de fixation étant situées entre les troisième et quatrième zones de fixation.

12. Article absorbant (100) selon l'une quelconque des revendications 8 à 11, dans lequel la troisième zone de fixation et la quatrième zone de fixation sont disposées symétriquement par rapport à une ligne centrale longitudinale de l'âme absorbante (130) s'étendant entre le premier et le second bord transversal.

13. Article absorbant (100) selon l'une quelconque des revendications précédentes, dans lequel la distance entre les première et deuxième zones de fixation est située entre 10 mm et 50 mm, préférablement entre 15 mm et 30 mm.

14. Article absorbant (100) selon l'une quelconque des revendications précédentes, dans lequel la longueur des première et deuxième zones de fixation est supérieure à 60 mm, préférablement supérieure à 70 mm.

15. Article absorbant (100) selon l'une quelconque des revendications précédentes, dans lequel le matériau absorbant comprend de la pâte cellulosique défibrée.

16. Article absorbant (100) selon l'une quelconque des revendications précédentes, dans lequel le matériau absorbant contient moins de 20 % en poids de pâte défibrée.

17. Article absorbant (100) selon l'une quelconque des revendications précédentes, dans lequel il n'y a pratiquement pas de matériau absorbant dans les première et deuxième zones de fixation.

18. Article absorbant (100) selon l'une quelconque des revendications précédentes, dans lequel les première et deuxième zones de fixation présentent chacune un fond et un dessus, la feuille d'enveloppe centrale supérieure étant fixée à la feuille d'enveloppe centrale inférieure au niveau dudit fond, au niveau dudit dessus ou entre ledit fond et ledit dessus.

19. Procédé de fabrication d'un article absorbant selon les revendications 1 à 18, ledit procédé comprenant :
a. le guidage d'un premier matériau en feuille (110) le long d'un élément rotatif (10), une surface (15) dudit élément rotatif étant pourvue d'un motif comportant des zones d'aspiration (13, 13') et des zones de non-aspiration (11, 12 ; 11', 12') ; lesdites zones de non-aspiration comprenant au moins des première et deuxième zones allongées s'étendant dans une direction circonférentielle de l'élément rotatif ;
b. l'application d'un matériau absorbant sur ledit premier matériau en feuille sur l'élément rotatif de telle sorte que les zones d'aspiration sont couvertes de matériau absorbant et qu'il n'y a pratiquement pas de matériau absorbant sur les zones de non-aspiration ;
c. appliquer un second matériau en feuille (120) sur le dessus du matériau absorbant sur le premier matériau en feuille ; l'un desdits premier et second matériaux en feuille étant un matériau en feuille d'enveloppe centrale supérieure et l'autre étant un matériau en feuille d'enveloppe centrale inférieure ;
d. la fixation dudit premier matériau en feuille (110) audit deuxième matériau en feuille (120) au moins dans les sections où il n'y a pratiquement pas de matériau absorbant, et de telle sorte qu'au moins des première et deuxième zones de fixation (140, 150) sont formées, situées à une certaine distance l'une de l'autre,
les première et deuxième zones de fixation étant parallèles dans une partie arrière de la région d'entrejambe, tandis que la distance transversale entre les première et deuxième zones de fixation augmente progressivement en direction du premier bord transversal de l'âme absorbante (130), le premier bord transversal étant un bord transversal avant de l'âme absorbante (130).

20. Procédé selon la revendication 19, dans lequel la fixation est effectuée par application de pression et de chaleur sur le matériau en feuille d'enveloppe centrale supérieure (110) et/ou sur le matériau en feuille d'enveloppe centrale inférieure dans les sections où il n'y a pratiquement pas de matériau absorbant.

21. Procédé selon la revendication 20, dans lequel la fixation est effectuée par un élément rotatif (30) qui est pourvu d'au moins une première et une deuxième nervure d'étanchéité (31, 32) dimensionnées pour appliquer une pression et de la chaleur sur le matériau en feuille d'enveloppe centrale supérieure (110) et/ou le matériau en feuille d'enveloppe centrale inférieure dans les sections où il n'y a pratiquement pas de matériau absorbant afin de créer les première et deuxième zones de fixation (140, 150), respectivement.
